# EUROPEAN PATENT SPECIFICATION

(11) **EP 1 025 083 B1**
(45) Date of publication and mention of the grant of the patent: **23.07.2003**
(21) Application number: 98953972.1
(22) Date of filing: 26.10.1998
(51) Int. Cl.: C07C 405/00

(54) **PROCESS FOR STEREOSELECTIVE SYNTHESIS OF PROSTACYCLIN DERIVATIVES**
VERFAHREN ZUR STEREOSELEKTIVEN SYNTHESE VON PROSTACYCLIN-DERIVATEN
PROCEDE DE SYNTHESE STEREOSELECTIVE DE DERIVES DE LA PROSTACYCLINE

(30) Priority: 24.10.1997 US 957736
(43) Date of publication of application: 09.08.2000
(73) Proprietor: United Therapeutics Corporation, Washington, DC 20009 (US)
(72) Inventor: MORIARTY, Robert, M., Oak Park, IL 60302 (US); PENMASTA, Raju, Bolingbrook, IL 60440 (US); GUO, Liang, Chicago, IL 60646 (US); RAO, Munagala, S., Westmont, IL 60559 (US); STASZEWSKI, James, P., Naperville, IL 60564 (US)
(74) Representative: VOSSIUS & PARTNER
(86) International application number: US9822585
(87) International publication number: WO99021830

(56) References cited:
- EP-A- 0 087 237
- WO-A-98/39337
- US-A- 4 306 075
- CHEMICAL ABSTRACTS, vol. 127, no. 5, 4 August 1997 Columbus, Ohio, US; abstract no. 65609, PAGENKOPF, BRIAN L.: "Substrate and reagent control of diastereoselectivity in transition metal-mediated processes: development of a catalytic photo promoted Pauson - Khand reaction" XP002097925 & (1996) 547 PP. AVAIL.: UNIV. MICROFILMS INT., ORDER NO. DA9717366 FROM: DISS. ABSTR. INT., B 1997, 57(12), 7535,
- MULZER, JOHANN ET AL: "Asymmetric synthesis of carbacyclin precursors by Pauson - Khand cyclization" LIEBIGS ANN. CHEM. (1988), (9), 891-7 CODEN: LACHDL;ISSN: 0170-2041, XP002097924

## Description

The present application relates to a process for producing prostacyclin derivatives and novel intermediate compounds useful in the process.

### BACKGROUND

Prostacyclin derivatives are useful pharmaceutical compounds possessing activities such as platelet aggregation inhibition, gastric secretion reduction, lesion inhibition, and bronchodilation.

For convenience, the novel prostacyclin derivatives wil be referred to by the trivial, art-recognized system of nomenclature described by N. A. Nelson, J. Med. Chem. 17:911 (1974) for prostaglandins. Accordingly, all of the novel prostacyclin derivatives herein will be named as 9- deoxy-PGP₁-type compounds.

U.S. Patent No. 4,306,075 discloses methods for making prostacyclin derivatives. However, these and other known processes involve a large number of steps. It is an object of the present invention to provide an improved method of preparing prostacyclin derivatives involving fewer steps.

### DETAILED DESCRIPTION

In one embodiment, the present invention relates to an improved stereoselective method for making 9-deoxy-PGF₁-type compounds comprising the following reaction: 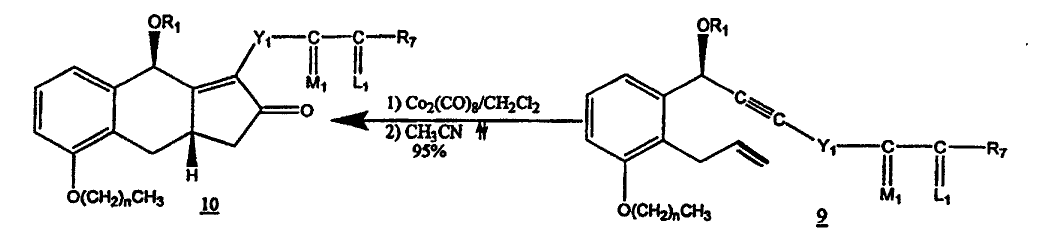 wherein n is 0, 1, 2, or 3;
wherein Y₁ is trans-CH=CH-, cis-CH=CH-, -CH₂(CH₂)ₘ-, or -C≡C-; m is 1,2, or 3;
wherein R1 is an alcohol protecting group;
wherein R₇ is
(1) -CₚH₂ₚ-CH₃, wherein p is an integer from one to 5, inclusive,
(2) phenoxy optionally substituted by one, two or three chloro, fluoro, trifluoromethyl, (C₁-C₃)alkyl, or (C₁-C₃)alkoxy, with the proviso that not more than two substituents are other than alkyl, with the proviso that R₇ is phenoxy or substituted phenoxy, only when R₃ and R₄ are hydrogen or methyl, being the same or different,
(3) phenyl, benzyl, phenylethyl, or phenylpropyl optionally substituted on the aromatic ring by one, two or three chloro, fluoro, trifluoromethyl, (C₁-C₃)alkyl, or (C₁-C₃)alkoxy, with the proviso that not more than two substituents are other than alkyl,
(4) cis-CH=CH-CH₂-CH₃,
(5) -(CH₂)₂-CH(OH)-CH₃, or
(6) -(CH₂)₃-CH=C(CH₃)₂;
wherein -C(L₁)-R₇ taken together is
(1) (C₄-C₇)cycloalkyl optionally substituted by one to 3 (C₁-C₅) alkyl;
(2) 2-(2-furyl)ethyl,
(3) 2-(3-thienyl)ethoxy, or
(4) 3-thienyloxymethyl;
wherein M₁ is α-OH:β-R₅ or α-R₅:β-OH, wherein R₅ is hydrogen or methyl;
wherein L₁ is α-R₃:β-R₄, α-R₄:β-R₃, or a mixture of α-R₃:β-R₄ and α-R₄:β-R₃, wherein R₃ and R₄ are hydrogen, methyl, or fluoro, being the same or different, with the proviso that one of R₃ and R₄ is fluoro only when the other is hydrogen or fluoro.

The present invention also relates to a method of making the following compounds utilizing the foregoing reaction: 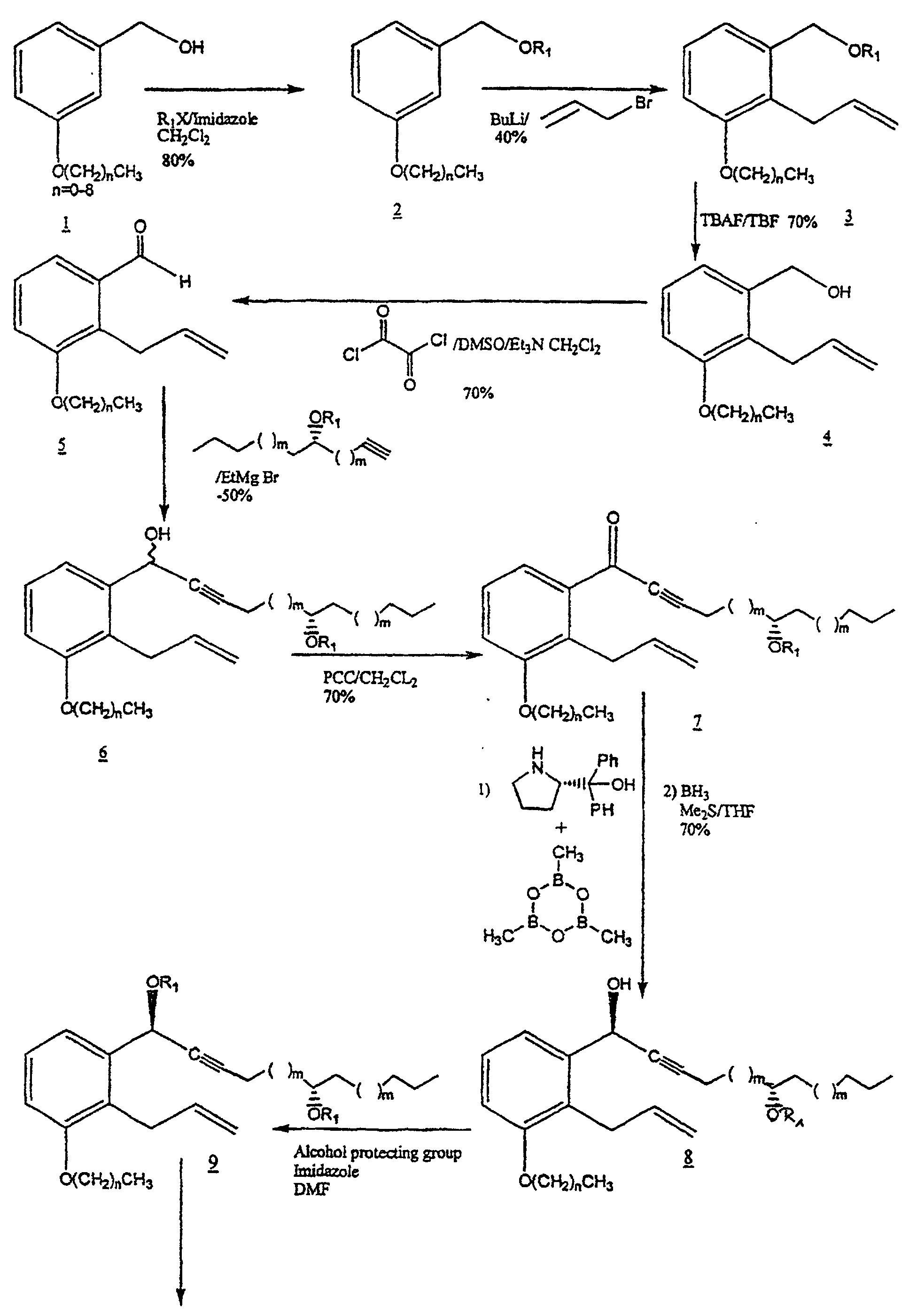 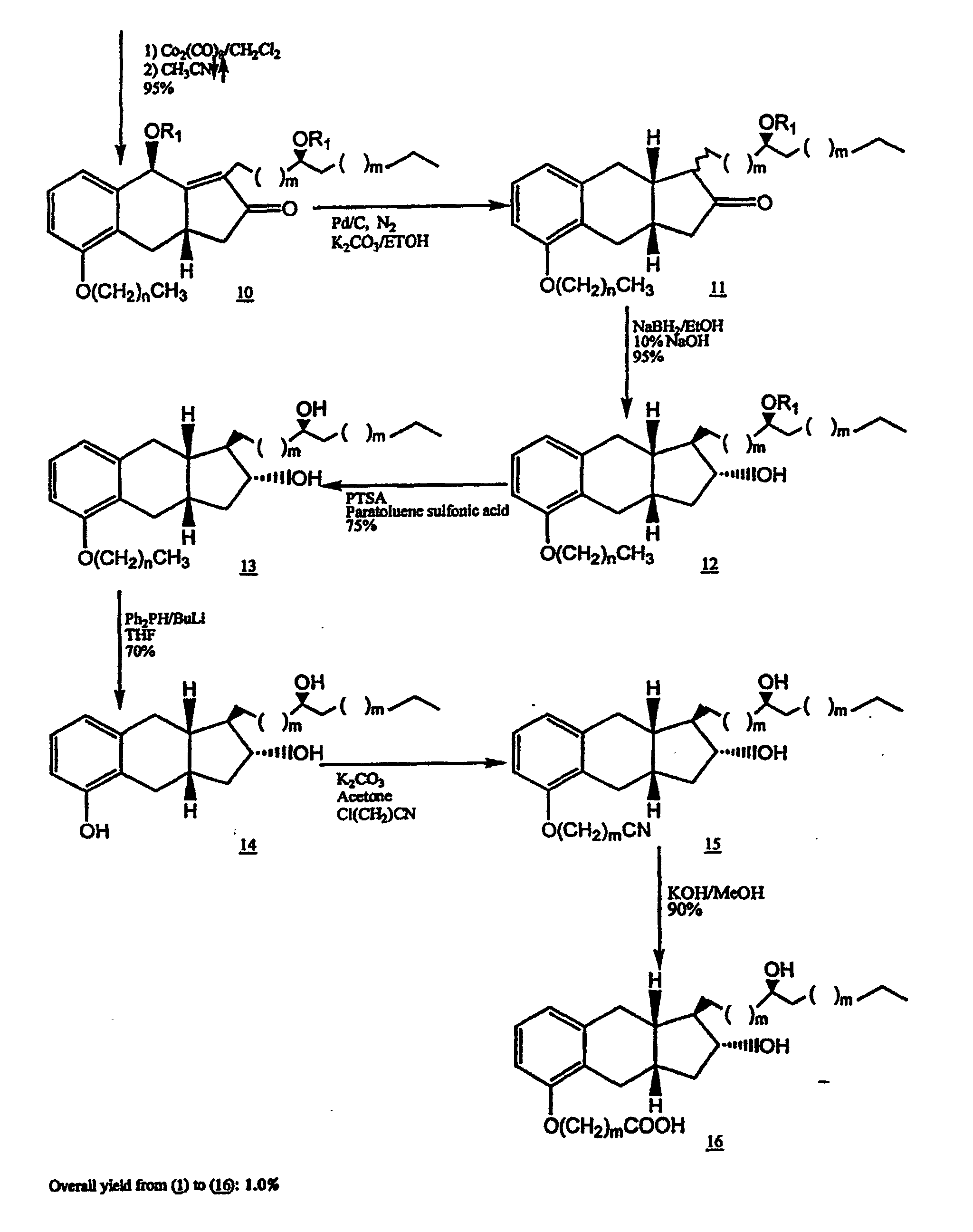 wherein R₁ is in each case an independently selected alcohol protecting group. Preferred alcohol protecting groups are tertiary butyl dimethyl sily (TBDMS) and tetra hydro pyranyl (THP).

The present invention also relates to the following novel intermediate compounds: 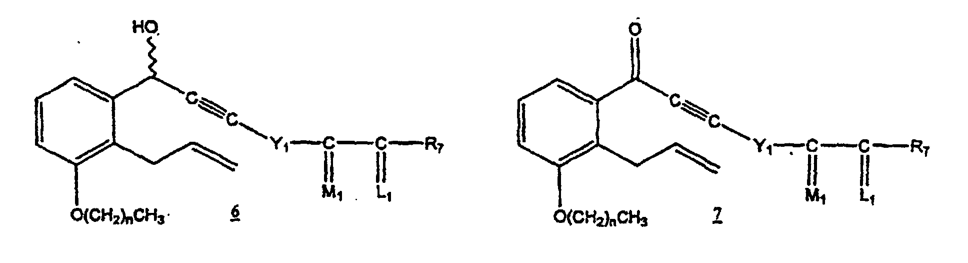 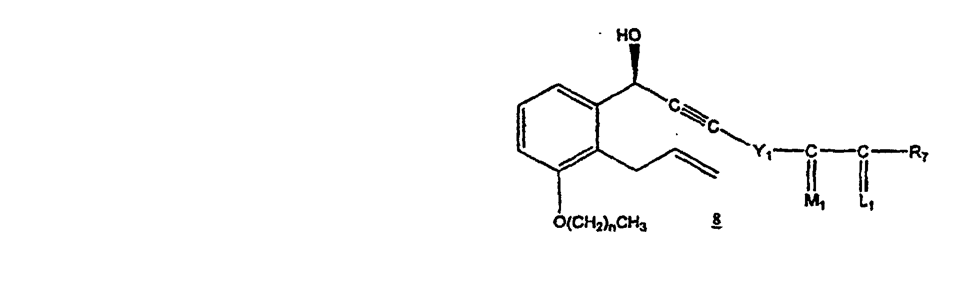 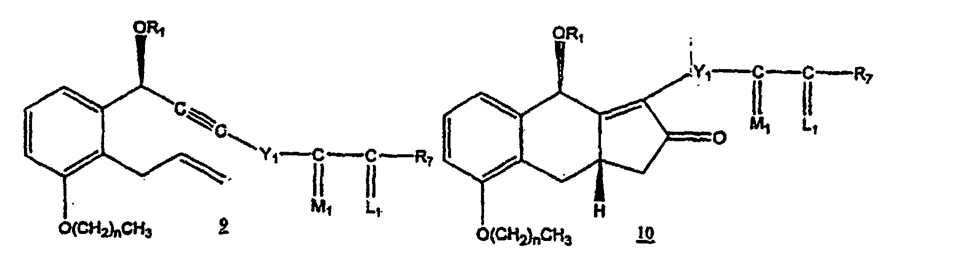 wherein Y₁, M₁, L₁, R₁ and R₇ are as defined above.

The present invention is further illustrated by, though in no way limited to, the following examples.

### Example 1

9-Deoxy-2',9α-methano-3-oxa-4,5,6-trinor-3,7-(1',3'-inter-phenylene)-13,14-dihydro-PGF₁

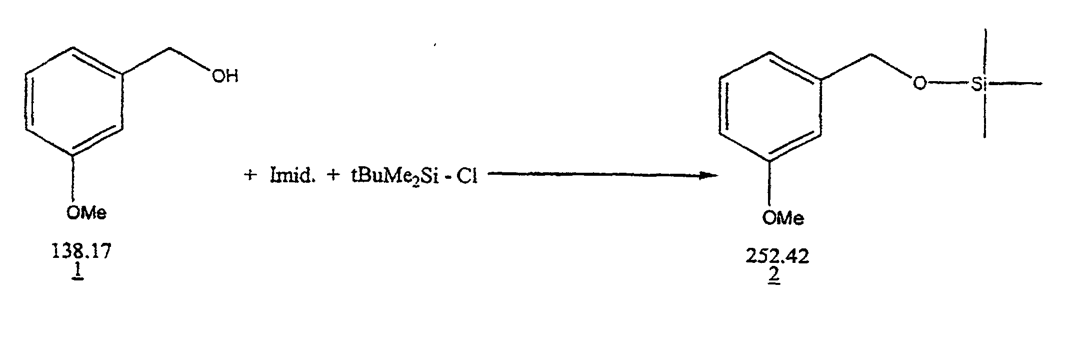

### Procedure

To a solution of imidazole (29.6g, 434 mmol, 2.8 eq.) in 1.0 L of methylene chloride were added 25 g (181 mmol) of 3-methoxybenzyl alcohol in 200 ml of methylene chloride. After all material was dissolved, 32.7 g (217 mmol, 1.2 eq.) of t-butyldimethylsilyl chloride were added in portions. The reaction was stirred overnight at room temperature. The mixture was filtered and washed with water and then brine. The organic layer was separated, dried over MgSO₄, filtered, and evaporated to afford 53 g of a clear yellow oil that was used in the next step without further purification.

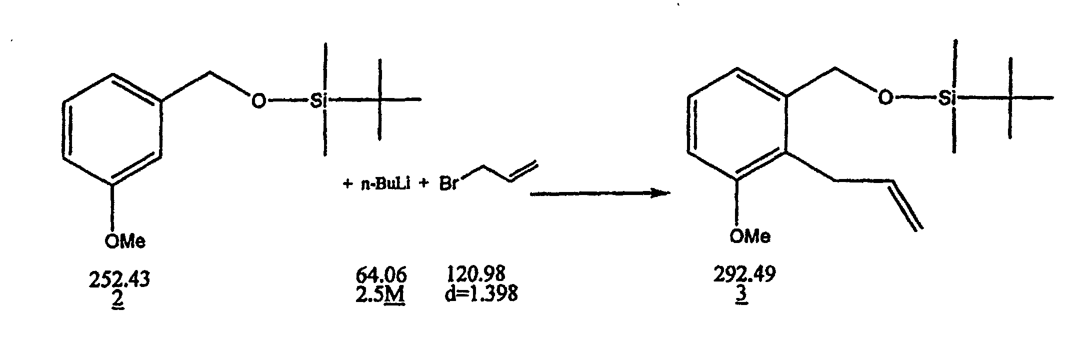

### Procedure

To a solution of 95 g (376 mmol) of **2** dissolved in 400 ml of hexane under Ar at room temperature were added dropwise 26.5 g (414 mmol, 1.1 eq.) of BuLi in 166 ml of hexane. The mixture was stirred for 2 hours at room temperature, and then the reaction was cooled in an ice bath and 54.6 g (452 mmol) of allyl bromide were added dropwise. The reaction was allowed to warm to room temperature overnight. After stirring for 24 hours, TLC indicated 60 % conversion, and the reaction was quenched with saturated NH₄Cl. The organic layer was separated and washed with Brine, dried over MgSO₄, and filtered. Evaporation of the solvent yielded a yellow oil which was used in the next reaction without further purification.

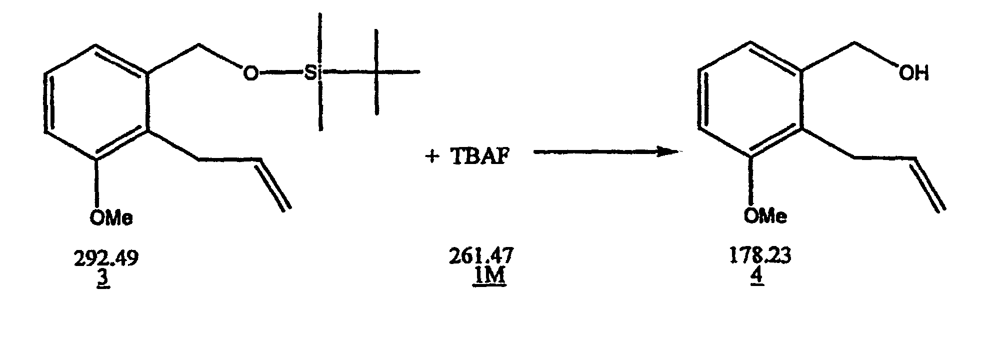

### Procedure

To a solution **3** (110 g, 376 mmol) in 2.0 L of THF were added 128 g (489 mmol, 1.1eq.) of tetrabutyl ammonium fluoride (TBAF) in 489 ml of THE. The reaction was stirred at room temperature and was complete after 4 hours. The reaction was quenched by adding 500 ml of water. The organic layer was separated and washed with brine and dried over MgSO₄. Filtration and evaporation of the solvent produced an orange oil which was purified by flash column chromatography, on silica gel using 10-30% ethyl acetate in hexanes as the eluent. The fractions containing the desired product were evaporated to afford 24 g (36 % from 3-methoxybenzyl alcohol) of a yellow oil.

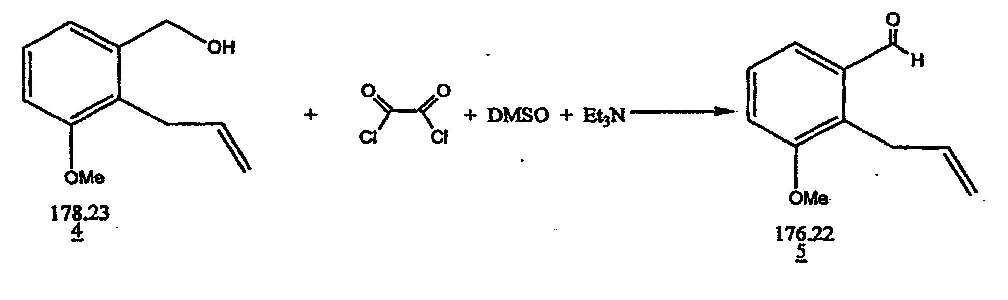

### Procedures

To a solution of 20.6 g (162 mmol, 1.2 eq.) of oxalyl chloride in 250 ml of CH₂Cl₂ under Ar at -78° C were added dropwise 24.2 g (310 mmol) of DMSO in 100 ml of CH₂Cl₂. After 10 minutes, 24 g (135 mmol) of **4** in 100 ml of CH₂Cl₂ were added dropwise. The mixture was stirred at -78° C for 30 min., and then 68.3g (675 mmol, 5.0 eq.) of Et₃N were added. Stirring continued as the reaction warmed to room temperature. The reaction was quenched with H₂O, washed with saturated NH₄Cl solution and Brine. The organic layer was separated and dried over MgSO₄. Filtration and evaporation of the solvent produced a brown oil which was purified by flash column chromatography, on silica gel using 5% ethyl acetate in hexanes as the eluent. The fractions containing the desired compound were evaporated to afford 20.5 g (86 %) of a brown oil.

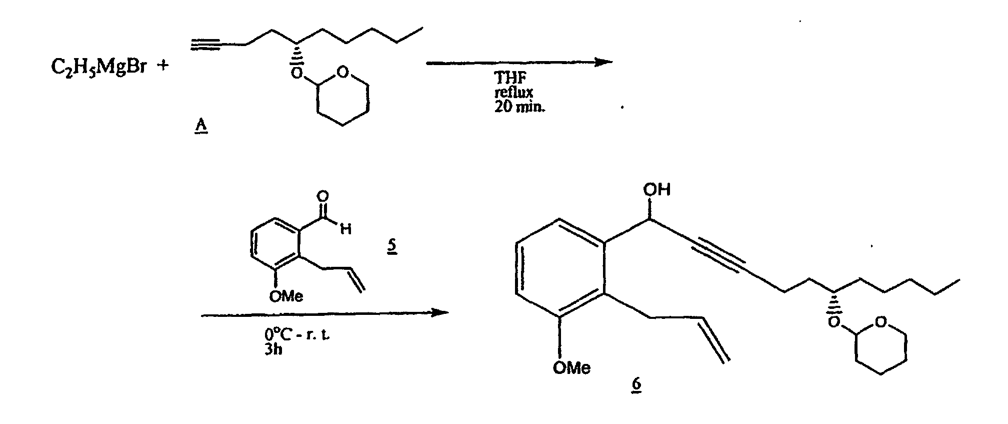

### Procedure

Compound A may be synthesized according to S. Takano et al., Chemistry Lett., 1987, p. 2017. To a solution of side chain (**A**) (1.6 g, 6.72 mmol) in dry THF (10 ml) which was heated to a gentle refluxing under argon was added EtMgBr (2.24 ml, 6.72 mmol, 3M solution). After the addition was complete, the resulting solution was refluxed for 20 min.

It was cooled to 0°C (under argon) and a solution of **5** (1.183 g, 6.72 mmol) in THF (10ml, dried over molecular sieves) was added dropwise with stirring. After the complete addition, the reaction mixture was allowed to warm to room temperature and stirred for 2-3 hrs. The reaction mixture was cooled to 0°C, diluted with saturated NH₄Cl solution, concentrated, extracted with ethyl acetate (4 x 25 ml), dried (MgSO₄) and the solvent distilled out *in vacuo*. The crude product (2.65 g) was purified by flash chromatography using 10-30% ether in hexane on silica gel to obtain a colorless oil 1.45 g (52%) of **6**.

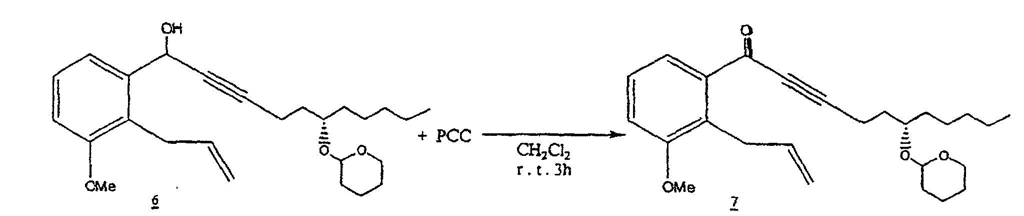

### Procedure

To a solution of alcohol **6** (1.27 g, 13.07 mmol) in dry CH₂Cl₂ (20 ml) was added pyridinium chlorochromate (PCC) (1.32 g, 6.12 mmol) and the mixture was stirred at room temperature. PCC slowly dissolved and the color of solution turned orange-black after approx. 5 min. Stirring was continued for 3 hrs. The reaction mixture was diluted with ether (100 ml) and filtered through a plug of silica gel. The solid was washed 3 times with ether (3 x 50 ml). After the solvent was removed, the crude product (1.3 g) was purified by flash chromatography using 10% ether in hexane on silica gel to give 900 mg light yellow oil (71%).

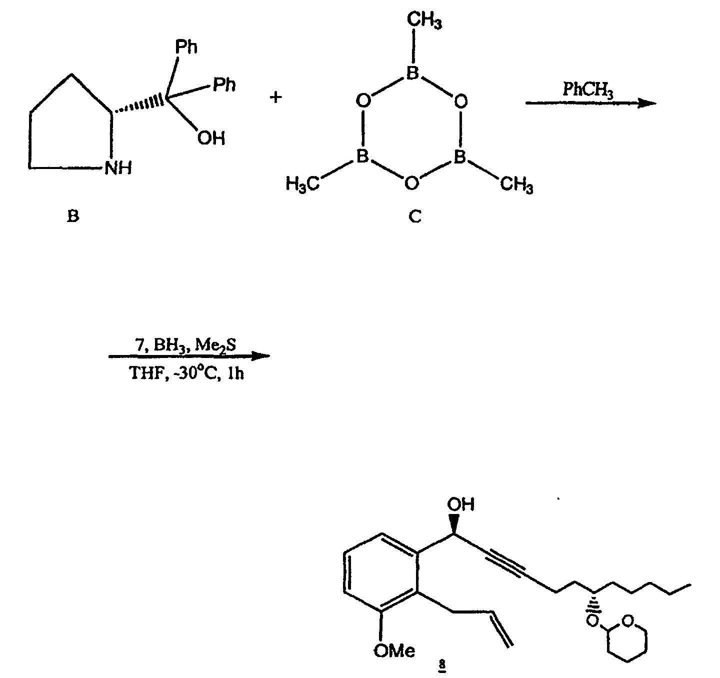

### Procedure

### STEP I: Preparation of Reagent :

Compound B may be synthesized according to D.S. Mathre et al., J. Org. Chem. 1991, Vol. 56, p. 751; P. Beak, Org. Synth., 1997, p. 23. Compound **B** (1.08 g, 4.26 mmol) was dissolved in 30 ml of anh. toluene under argon. Trimethylboroxine (**C**) (0.357 g, 2.84 mmol) was added dropwise and the resulting solution was stirred at room temperature. White solid separated out after 3-4 min. After stirring for 30 min., toluene was distilled out at atmospheric pressure. Again 20 ml of dry toluene were added and distilled out. This distillation was repeated for 2 more times. The solution of reagent in toluene was allowed to cool under argon.

### STEP II: Reduction :

A solution of ketone **7** ( 0.88 g, 2.14 mmol) in dry THF (20 ml) was dried over molecular sieves for 2 hrs and added to the above reagent solution. The resulting solution was cooled to -30°C (CH₃CN, CO₂) under argon and borane-methylsulfide complex (1.07 ml, 10.71 mmol) was added dropwise with stirring. After stirring at -30°C for 1 hr, the reaction was quenched with methanol (10 ml), diluted with ether (100 ml), washed successively with saturated NH₄Cl, NaHCO₃ solution and brine, dried (MgSO₄) and concentrated *in vacuo* to yield a crude product ( 2.3 g). The crude product was purified by flash chromatography using 10% ether in hexane on silica gel to give 770 mg of 8 as a colorless oil (87%).

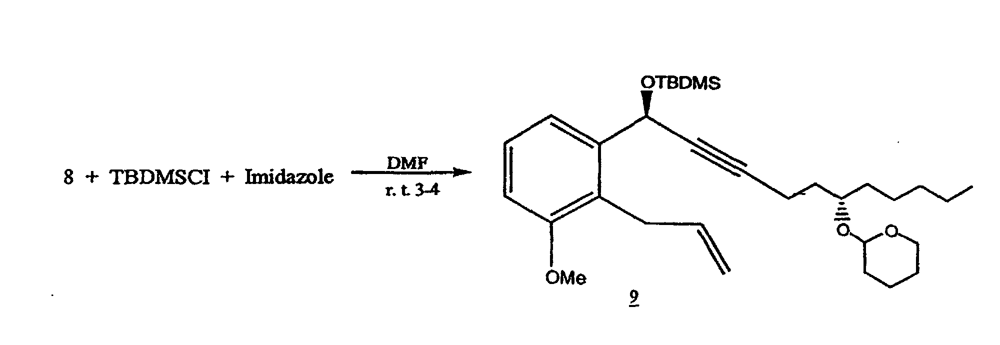

### Procedure

TBDMSC1 (0.337 g, 2.23 mmol) and imidazole (0.335 g, 4.65 mmol) were added to the solution of **8** (0.770 g, 1.86 mmol) in DMF (20 ml) at room temperature under argon, and the mixture was stirred at room temperature for 3-4 hrs. After the reaction was quenched with sat. NH₄Cl, the reaction mixture was extracted with ether (3 x 50 ml). The combined ether extracts were dried (MgSO₄) and concentrated *in vacuo*. The crude oil was purified by chromatography using 5% ether in hexane on silica gel to yield 860 mg of **9** as a colorless oil (88%).

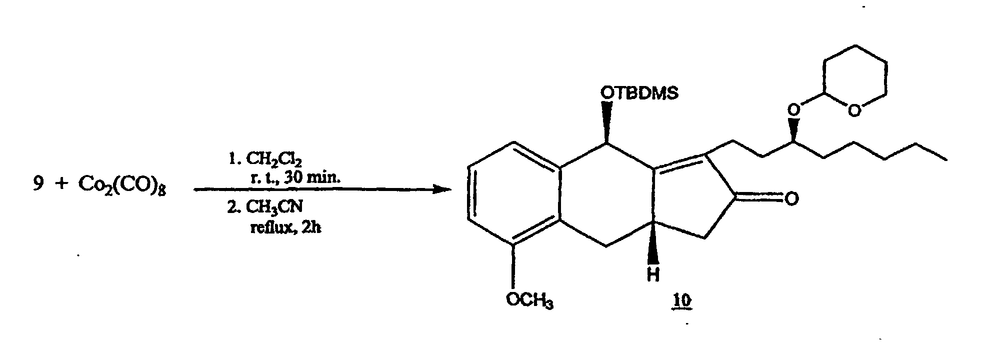

### Procedure

### STEP I : Complex formation:

Compound **9** (0.840 g, 1.59 mmol) was dissolved in dry CH₂Cl₂ (15ml) under argon, and Co₂(CO)₈ (0.653 g, 1.91 mmol) was added to it and stirred at room temperature under argon. carbon monoxide evolved out slowly, and the solution turned dark brown after 5 min. Stirring was continued for 30 min. at room temperature.

### STEP II: Pauson Khand Cyclization

CH₂Cl₂ was distilled out from the above solution. The complex was dissolved in dry CH₃CN (50 ml), and the solution was refluxed under argon for 2 hrs. This solvent was distilled out, the crude mass was dissolved in ether and passed quickly through a short column of neutral alumina to yield 850 mg of light brown oil (96%).

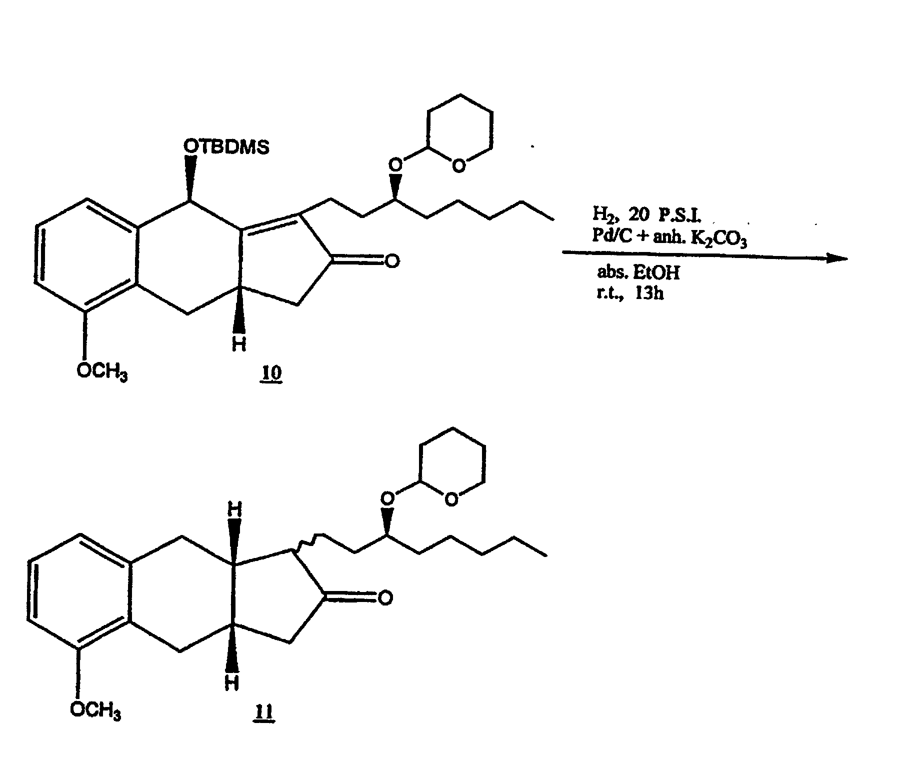

### Procedure

Compound **10** (0.850 g, 1.53 mmol) was dissolved in absolute ethanol (50 ml). Anh. K₂CO₃ (0.020 g) and Pd/C (0.550 g, 10%, wet) were added and the mixture was hydrogenated at 20 psi pressure for 13 hrs. The reaction mixture was filtered through celite and concentrated *in vacuo*. The crude product (800 mg) was purified by chromatography using 10-30% ether in hexane on silica gel to yield 440 mg of colorless oil (67%).

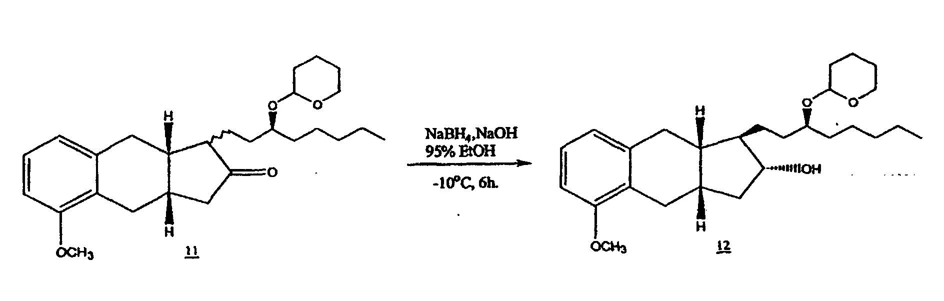

### Procedure

A solution of ketone **11** (0.430 g) in 95% ethanol was cooled to -10°C. 10% NaOH (6 ml) and NaBH₄ (0.080 g) were added and the mixture was stirred at -10°C for 1hr. Then one more eq. of NaBH_{4 (0.080 g)} was added and stirring was continued for another 5 hrs. at -10°C. After quenching carefully with glacial acetic acid, the solvent was removed under reduced pressure. Resulting oil was dissolved in ethyl acetate, washed with aq. NaHCO₃, brine, dried (MgSO₄) and concentrated *in vacuo* to obtain 430 mg of colorless oil (98%) which has a single spot on TLC. Further purification was not required.

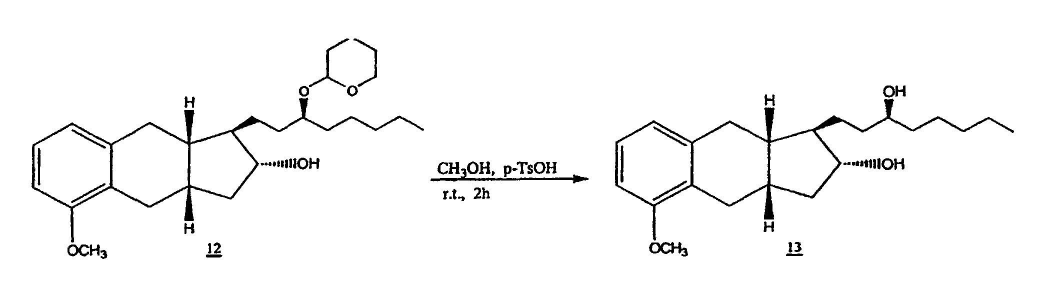

### Procedure

To 400 mg (0.93 mmol) of compound **12** dissolved in methanol (10 ml) was added p-TsOH (20 mg), and the solution was stirred at room temperature until TLC showed completion of the reaction (2 hrs). The solvent was removed *in vacuo*, the residue was dissolved in CH₂Cl₂, washed with sat. NaHCO₃, dried(MgSO₄), and concentrated *in vacuo*. The crude product was purified by silica gel column chromatography ( 30% ether in hexanes as eluent) to give 250 mg **13** (78%).

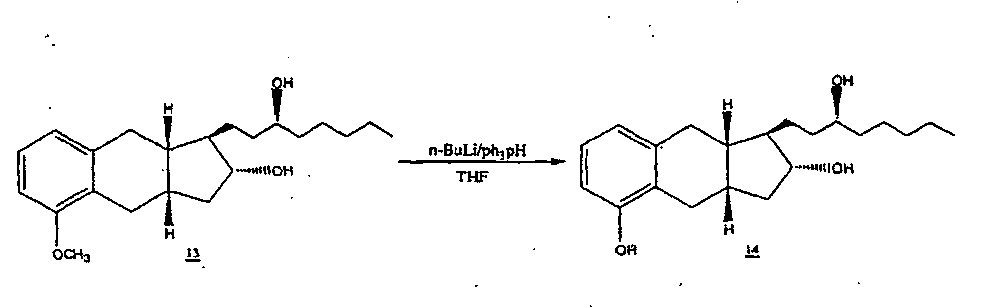

### Procedure

n-BuLi (1.1 ml, 1.72mmol)(1.6 M in hexanes) was added dropwise to a cold (-20°C) and stirred solution of diphenylphosphine (0.28 g, 1.5 mmol) in anhydrous THF (8 ml) under argon. The reaction mixture was warmed to room temperature (20°C). A solution of diol (**13**) (0.17 g, 0.49 mmol) in dry THF (0.6 ml) was added dropwise to the reaction mixture and the whole solution was heated to reflux for 3 hrs (TLC shows starting material), heating was stopped and the reaction mixture was cooled again to -20°C and diphenylphosphine (0.37 g, 1.96 mmol) was added followed by dropwise addition of n-BuLi (1.5 ml, 2.38 mmol)(1.6M in hexanes) under argon. After complete addition, the reaction mixture was warmed to 20°C and then refluxed for 18 hrs. TLC shows 80-90% conversion (**14**). The reaction mixture was cooled to -5°C and then an aqueous solution of NaCl containing 5% cone. HCl was added dropwise to quench the reaction. The reaction mixture was extracted with ethyl acetate 3x20 ml and the combined organic layers were washed with brine and dried (Na₂SO₄), filtered and concentrated. The crude product was purified by silica gel column chromatography (50% EtOAc/Hex, as eluent) to give 0.12 g of product (75%) (22 mg of starting diol was recovered).

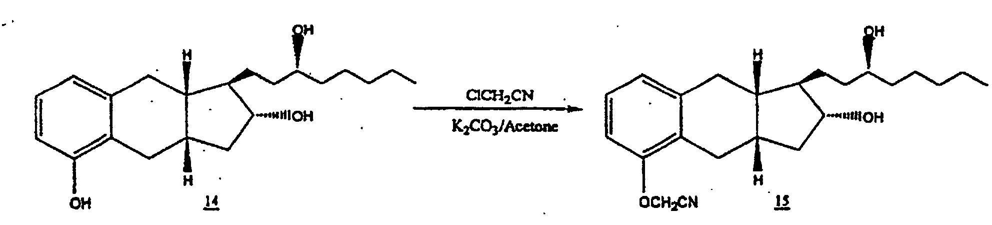

### Procedure

A suspension of compound (**14**) (0.12 g. 0.37 mmol), chloroacetonitrile (0.56 g, 7.4 mmol) and K₂CO₃ (0.51 g, 3.7 mmol) in dry acetone (15 ml) was refluxed under Ar for 20 hrs. The reaction mixture was cooled to room temperature and celite (0.5 g) was added. After the mixture was filtered, the solvent was removed under reduced pressure. The crude product was purified by silica gel column chromatography using 1:1 EtOAc/hexanes as eluent to yield 0.12 g of product (95%).

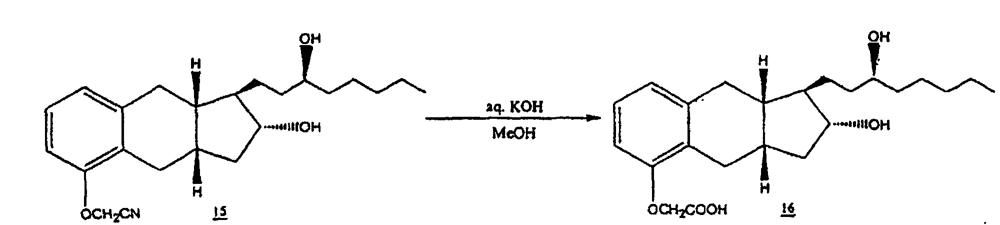

### Procedure

Aqueous KOH (0.4 g, 7.12 mmol. water 1.2 ml, 35% solution) was added dropwise to a stirred solution of nitrite compound (**15**) (0.072 g, 0.21 mmol) in methanol (4 ml) and the reaction mixture was refluxed for 3 hrs. The reaction mixture was cooled to 10°C, dilute aqueous HCl was added to pH 8 and the solvent was removed *in vacuo*. Ethyl acetate (20 ml) and aqueous NaCl solution (10 ml) were added and the pH of the reaction mixture was acidified to between 2 and 3 by addition of 2% HCl. The reaction mixture was extracted with ethyl acetate (2x20 ml). The combined ethyl acetate extracts were washed with brine, dried (Na₂SO₄) and concentrated under reduced pressure. The crude product was purified by silica gel column chromatography using a dichloromethane solution containing 3% methanol and 0.1% acetic acid as eluent to yield 0.076 g of product (95%).

It will be apparent to those skilled in the art that various modifications and variations can be made to the processes and novel intermediates of this invention. Thus, it is intended that the present invention cover such modifications and variations, provided they come within the scope of the appended claims and their equivalents.

The disclosure of all publications cited above are expressly incorporated herein by reference in their entireties to the same extent as if each were incorporated by reference individually.

## Claims

1. A stereoselective method of making a 9-deoxy-PGF₁-type compound, comprising the following reaction: 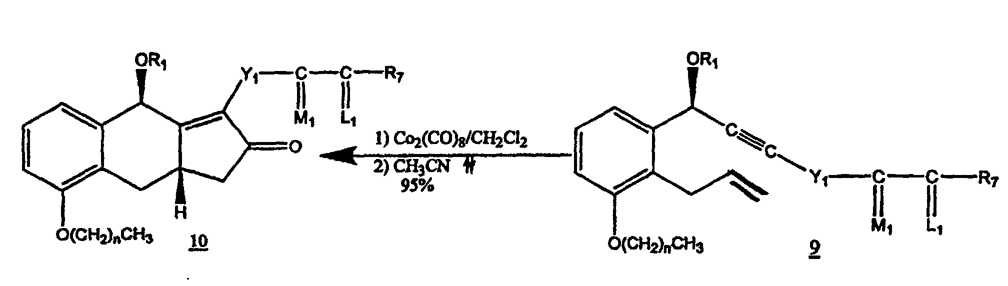 wherein R₁ is an alcohol protecting group;
wherein n is 0, 1, 2, or 3;
wherein Y₁ is trans-CH=CH-, cis-CH=CH-, -CH₂(CH₂)ₘ-, or -C≡C-; m is 1,2, or 3;
wherein R₇ is
(1) -CₚH₂ₚ-CH₃, wherein p is an integer from one to 5, inclusive,
(2) phenoxy optionally substituted by one, two or three chloro, fluoro, trifluoromethyl, (C₁-C₃)alkyl, or (C₁-C₃)alkoxy, with the proviso that not more than two substituents are other than alkyl, with the proviso that R₇ is phenoxy or substituted phenoxy, only when R₃ and R₄ are hydrogen or methyl, being the same or different,
(3) phenyl, benzyl, phenylethyl, or phenylpropyl optionally substituted on the aromatic ring by one, two or three chloro, fluoro, trifluoromethyl, (C₁-C₃)alkyl, or (C₁-C₃)alkoxy, with the proviso that not more than two substituents are other than alkyl,
(4) cis-CH=CH-CH₂-CH₃,
(5) -(CH₂)₂-CH(OH)-CH₃, or
(6) -(CH₂)₃-CH=C(CH₃)₂;
wherein -C(L₁)-R₇ taken together is
(1) (C₄-C₇)cycloalkyl optionally substituted by one to 3 (C₁-C₅) alkyl;
(2) 2-(2-furyl)ethyl,
(3) 2-(3-thienyl)ethoxy, or
(4) 3-thienyloxymethyl;
wherein M₁ is α-OH:β-R₅ or α-R₅:β-OH, wherein R₅ is hydrogen or methyl;
wherein L₁ is α-R₃:β-R₄, α-R₄:β-R₃, or a mixture of α-R₃:β-R₄ and α-R₄:β-R₃, wherein R₃ and R₄ are hydrogen, methyl, or fluoro, being the same or different, with the proviso that one of R₃ and R₄ is fluoro only when the other is hydrogen or fluoro.

2. The method as claimed in claim 1, further comprising the following steps: 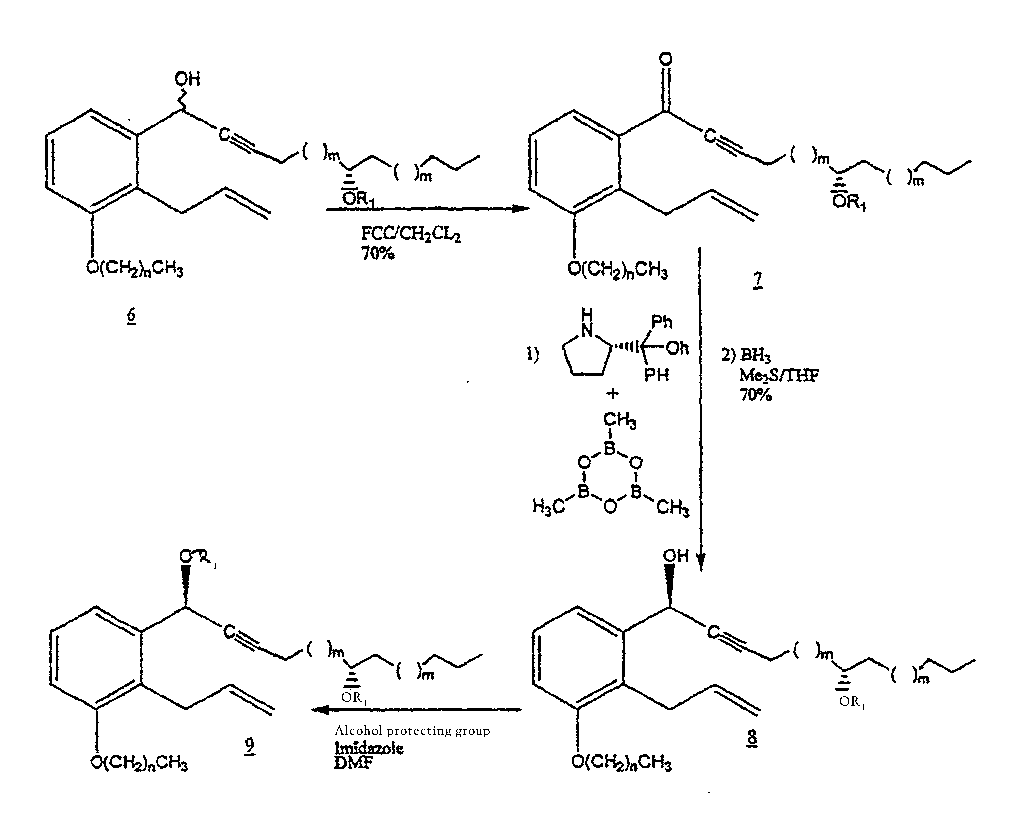 wherein R₁ is an independently selected alcohol protecting group, m is 1, 2, or 3 and n is 0, 1, 2, or 3.

3. The method as claimed in claim 2, comprising the following steps: 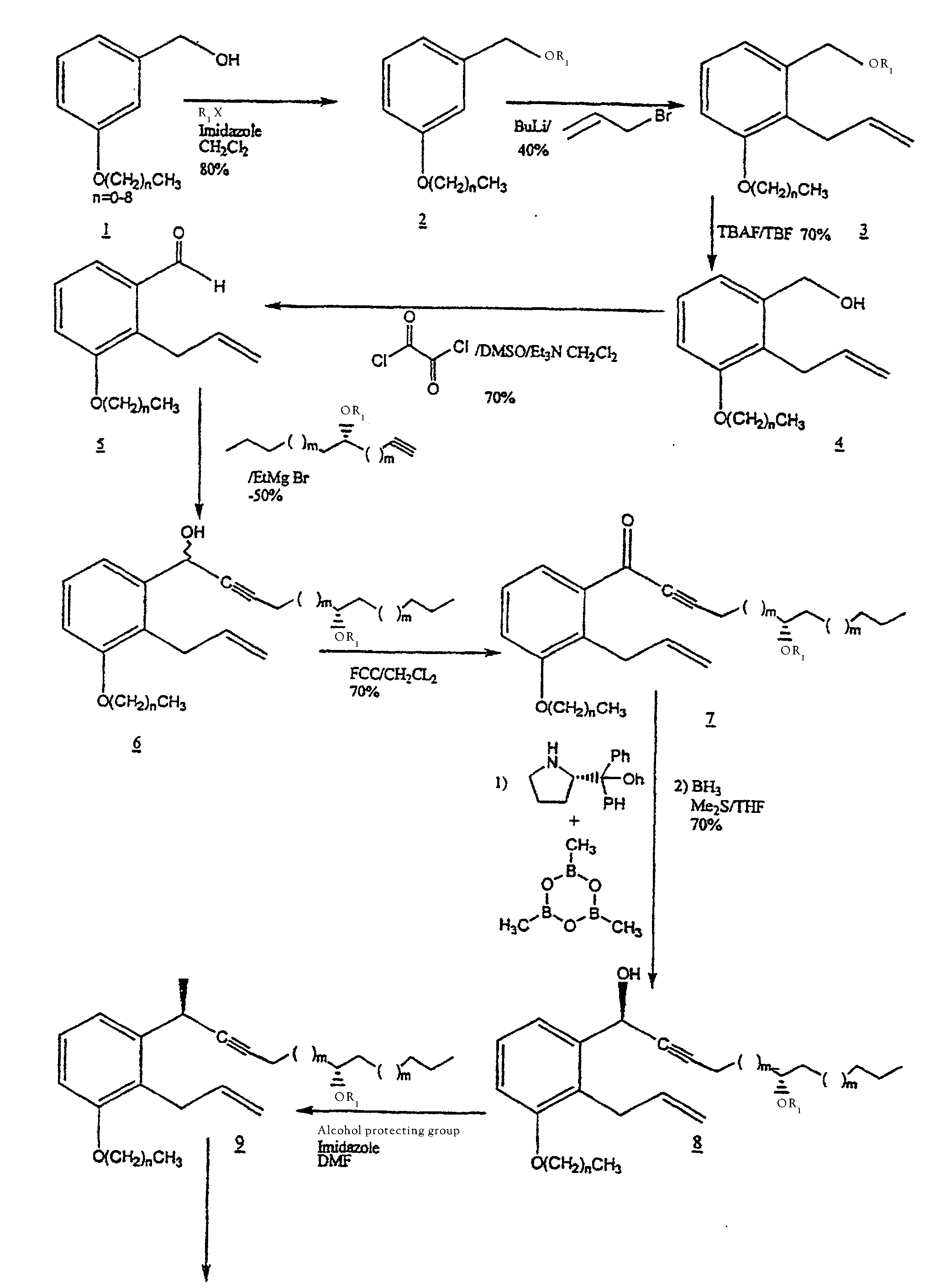 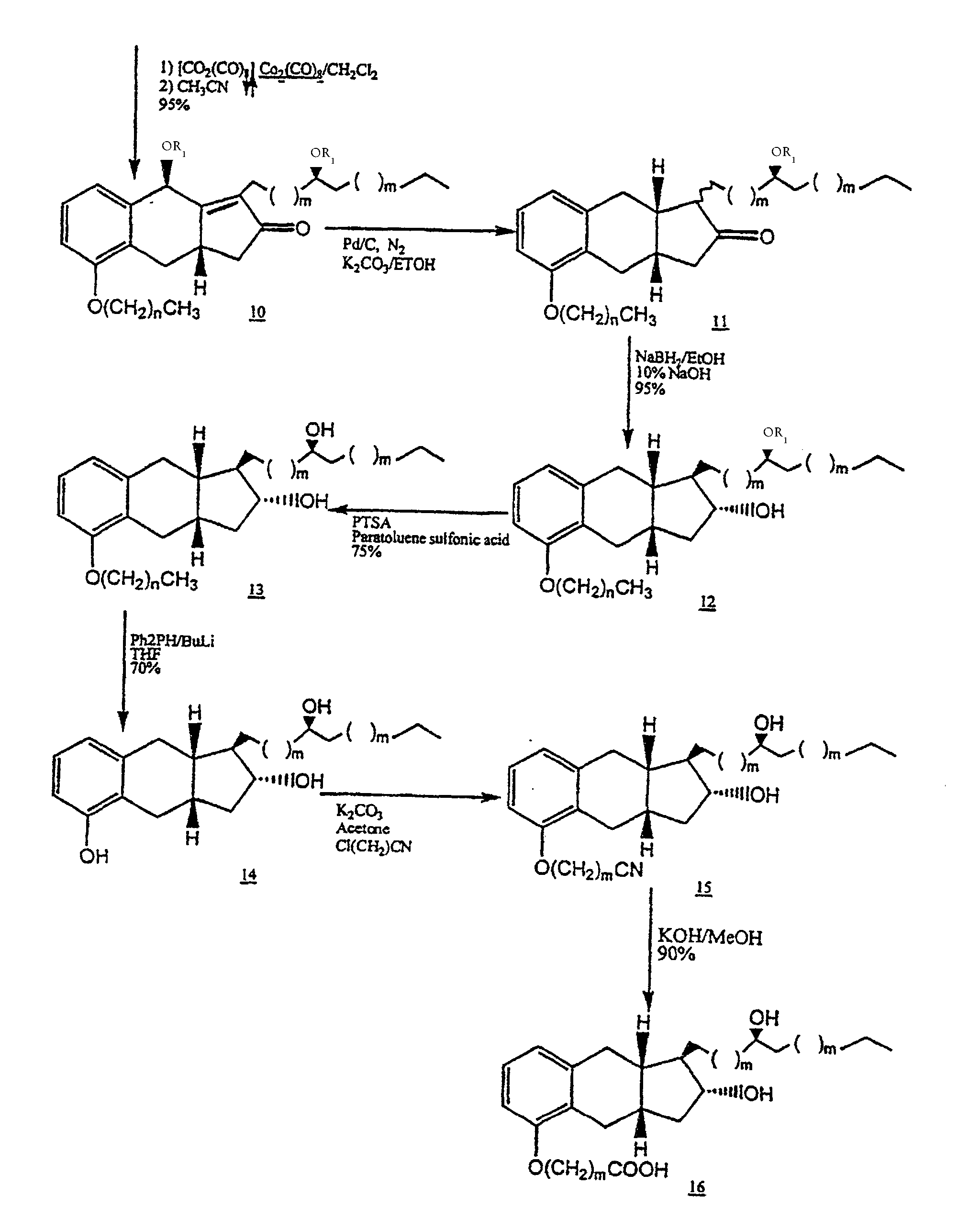 wherein R_{1,} m and n are the same as defined in claim 2.

4. The method as claimed in claim 3 wherein m is 1 and n is 0.

5. A compound of the formula: 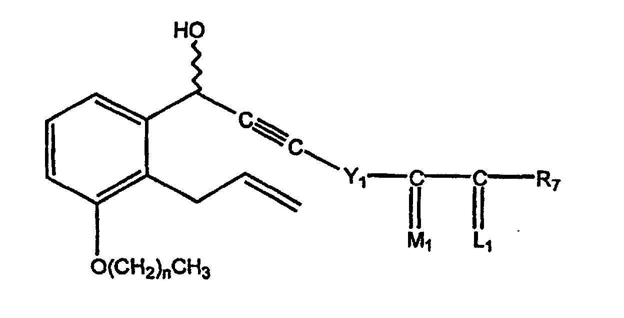 wherein Y₁ is trans-CH=CH-, cis-CH=CH-, -CH₂(CH₂)ₘ-, or -C≡C-; m is 1, 2, or 3;
wherein n is 0, 1, 2, or 3;
wherein R₇ is
(1) -CₚH₂ₚ-CH₃, wherein p is an integer from one to 5, inclusive,
(2) phenoxy optionally substituted by one, two or three chloro, fluoro, trifluoromethyl, (C₁-C₃)alkyl, or (C₁-C₃)alkoxy, with the proviso that not more than two substituents are other than alkyl, with the proviso that R₇ is phenoxy or substituted phenoxy, only when R₃ and R₄ are hydrogen or methyl, being the same or different,
(3) phenyl, benzyl, phenylethyl, or phenylpropyl optionally substituted on the aromatic ring by one, two or three chloro, fluoro, trifluoromethyl, (C₁-C₃)alkyl, or (C₁-C₃)alkoxy, with the proviso that not more than two substituents are other than alkyl,
(4) cis-CH=CH-CH₂-CH₃,
(5) -(CH₂)₂-CH(OH)-CH₃, or
(6) -(CH₂)₃-CH=C(CH₃)₂;
wherein -C(L₁)-R₇ taken together is
(1) (C₄-C₇)cycloalkyl optionally substituted by one to 3 (C₁-C₅) alkyl;
(2) 2-(2-furyl)ethyl,
(3) 2-(3-thienyl)ethoxy, or
(4) 3-thienyloxymethyl;
wherein M₁ is α-OH:β-R₅ or α-R₅:β-OH, wherein R₅ is hydrogen or methyl;
wherein L₁ is α-R₃:β-R₄, α-R₄:β-R₃, or a mixture of α-R₃:β-R₄ and α-R₄:β-R₃, wherein R₃ and R₄ are hydrogen, methyl, or fluoro, being the same or different, with the proviso that one of R₃ and R₄ is fluoro only when the other is hydrogen or fluoro.

6. A compound of the formula: 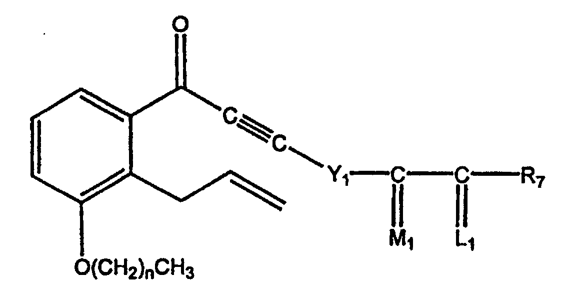 wherein Y₁ is trans-CH=CH-, cis-CH=CH-, -CH₂(CH₂)ₘ-, or -C≡C-; m is 1,2, or 3;
wherein n is 0, 1, 2, or 3;
wherein R₇ is
(1) -CₚH₂ₚ-CH₃, wherein p is an integer from one to 5, inclusive,
(2) phenoxy optionally substituted by one, two or three chloro, fluoro, trifluoromethyl, (C₁-C₃)alkyl, or (C₁-C₃)alkoxy, with the proviso that not more than two substituents are other than alkyl, with the proviso that R₇ is phenoxy or substituted phenoxy, only when R₃ and R₄ are hydrogen or methyl, being the same or different,
(3) phenyl, benzyl, phenylethyl, or phenylpropyl optionally substituted on the aromatic ring by one, two or three chloro, fluoro, trifluoromethyl, (C₁-C₃)alkyl, or (C₁-C₃)alkoxy, with the proviso that not more than two substituents are other than alkyl,
(4) cis-CH=CH-CH₂-CH₃,
(5) -(CH₂)₂-CH(OH)-CH₃, or
(6) -(CH₂)₃-CH=C(CH₃)₂;
wherein -C(L₁)-R₇ taken together is
(1) (C₄-C₇)cycloalkyl optionally substituted by one to 3 (C₁-C₅) alkyl;
(2) 2-(2-furyl)ethyl,
(3) 2-(3-thienyl)ethoxy, or
(4) 3-thienyloxymethyl;
wherein M₁ is α-OH:β-R₅ or α-R₅:β-OH, wherein R₅ is hydrogen or methyl;
wherein L₁ is α-R₃:β-R₄, α-R₄:β-R₃, or a mixture of α-R₃:β-R₄ and α-R₄:β-R₃, wherein R₃ and R₄ are hydrogen, methyl, or fluoro, being the same or different, with the proviso that one of R₃ and R₄ is fluoro only when the other is hydrogen or fluoro.

7. A compound of the formula 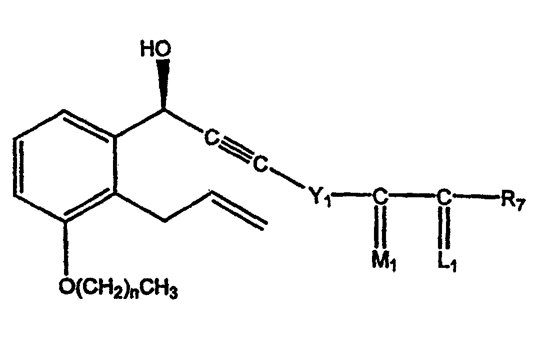 wherein Y₁ is trans-CH=CH-, cis-CH=CH-, -CH₂(CH₂)ₘ-, or -C≡C-; m is 1,2, or 3;
wherein n is 0, 1, 2, or 3;
wherein R₇ is
(1) -CₚH₂ₚ-CH₃, wherein p is an integer from one to 5, inclusive,
(2) phenoxy optionally substituted by one, two or three chloro, fluoro, trifluoromethyl, (C₁-C₃)alkyl, or (C₁-C₃)alkoxy, with the proviso that not more than two substituents are other than alkyl, with the proviso that R₇ is phenoxy or substituted phenoxy, only when R₃ and R₄ are hydrogen or methyl, being the same or different,
(3) phenyl, benzyl, phenylethyl, or phenylpropyl optionally substituted on the aromatic ring by one, two or three chloro, fluoro, trifluoromethyl, (C₁-C₃)alkyl, or (C₁-C₃)alkoxy, with the proviso that not more than two substituents are other than alkyl,
(4) cis-CH=CH-CH₂-CH₃,
(5) -(CH₂)₂-CH(OH)-CH₃, or
(6) -(CH₂)₃-CH=C(CH₃)₂;
wherein -C(L₁)-R₇ taken together is
(1) (C₄-C₇)cycloalkyl optionally substituted by one to 3 (C₁-C₅) alkyl;
(2) 2-(2-furyl)ethyl,
(3) 2-(3-thienyl)ethoxy, or
(4) 3-thienyloxymethyl;
wherein M₁ is α-OH:β-R₅ or α-R₅:β-OH, wherein R₅ is hydrogen or methyl;
wherein L₁ is α-R₃:β-R₄, α-R₄:β-R₃, or a mixture of α-R₃:β-R₄ and α-R₄:β-R₃, wherein R₃ and R₄ are hydrogen, methyl, or fluoro, being the same or different, with the proviso that one of R₃ and R₄ is fluoro only when the other is hydrogen or fluoro.

8. A compound of the formula 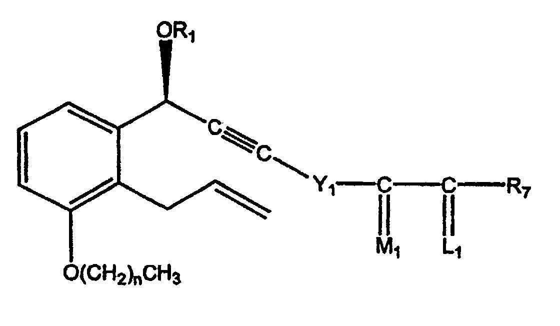 wherein R₁ is an alcohol protecting group;
wherein n is 0, 1, 2, or 3;
wherein Y₁ is trans-CH=CH-, cis-CH=CH-, -CH₂(CH₂)ₘ-, or -C≡C-; m is 1,2, or 3;
wherein R₇ is
(1) -CₚH₂ₚ-CH₃, wherein p is an integer from one to 5, inclusive,
(2) phenoxy optionally substituted by one, two or three chloro, fluoro, trifluoromethyl, (C₁-C₃)alkyl, or (C₁-C₃)alkoxy, with the proviso that not more than two substituents are other than alkyl, with the proviso that R₇ is phenoxy or substituted phenoxy, only when R₃ and R₄ are hydrogen or methyl, being the same or different,
(3) phenyl, benzyl, phenylethyl, or phenylpropyl optionally substituted on the aromatic ring by one, two or three chloro, fluoro, trifluoromethyl, (C₁-C₃)alkyl, or (C₁-C₃)alkoxy, with the proviso that not more than two substituents are other than alkyl,
(4) cis-CH=CH-CH₂-CH₃,
(5) -(CH₂)₂-CH(OH)-CH₃, or
(6) -(CH₂)₃-CH=C(CH₃)₂;
wherein -C(L₁)-R₇ taken together is
(1) (C₄-C₇)cycloalkyl optionally substituted by one to 3 (C₁-C₅) alkyl;
(2) 2-(2-furyl)ethyl,
(3) 2-(3-thienyl)ethoxy, or
(4) 3-thienyloxymethyl;
wherein M₁ is α-OH:β-R₅ or α-R₅:β-OH, wherein R₅ is hydrogen or methyl;
wherein L₁ is α-R₃:β-R₄, α-R₄:β-R₃, or a mixture of α-R₃:β-R₄ and α-R₄:β-R₃, wherein R₃ and R₄ are hydrogen, methyl, or fluoro, being the same or different, with the proviso that one of R₃ and R₄ is fluoro only when the other is hydrogen or fluoro.

9. A compound of the formula 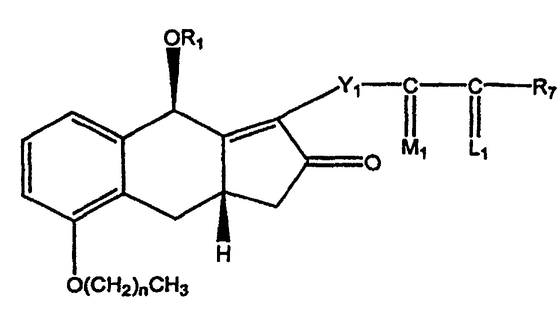 wherein R₁ is an alcohol protecting group;
wherein n is 0, 1, 2, or 3;
wherein Y₁ is trans-CH=CH-, cis-CH=CH-, -CH₂(CH₂)ₘ-, or -C≡C-; m is 1, 2, or 3;
wherein R₇ is
(1) -CₚH₂ₚ-CH₃, wherein p is an integer from one to 5, inclusive,
(2) phenoxy optionally substituted by one, two or three chloro, fluoro, trifluoromethyl, (C₁-C₃)alkyl, or (C₁-C₃)alkoxy, with the proviso that not more than two substituents are other than alkyl, with the proviso that R₇ is phenoxy or substituted phenoxy, only when R₃ and R₄ are hydrogen or methyl, being the same or different,
(3) phenyl, benzyl, phenylethyl, or phenylpropyl optionally substituted on the aromatic ring by one, two or three chloro, fluoro, trifluoromethyl, (C₁-C₃)alkyl, or (C₁-C₃)alkoxy, with the proviso that not more than two substituents are other than alkyl,
(4) cis-CH=CH-CH₂-CH₃,
(5) -(CH₂)₂-CH(OH)-CH₃, or
(6) -(CH₂)₃-CH=C(CH₃)₂;
wherein -C(L₁)-R₇ taken together is
(1) (C₄-C₇)cycloalkyl optionally substituted by one to 3 (C₁-C₃) alkyl;
(2) 2-(2-furyl)ethyl,
(3) 2-(3-thienyl)ethoxy, or
(4) 3-thienyloxymethyl;
wherein M₁ is α-OH:β-R₅ or α-R₅:β-OH, wherein R₅ is hydrogen or methyl;
wherein L₁ is α-R₃:β-R₄, α-R₄:β-R₃, or a mixture of α-R₃:β-R₄ and α-R₄:β-R₃, wherein R₃ and R₄ are hydrogen, methyl, or fluoro, being the same or different, with the proviso that one of R₃ and R₄ is fluoro only when the other is hydrogen or fluoro.

## Patentansprüche

1. Stereoselektives Verfahren zur Herstellung einer Verbindung vom 9-Desoxy-PGF₁-Typ, umfassend die folgende Reaktion: 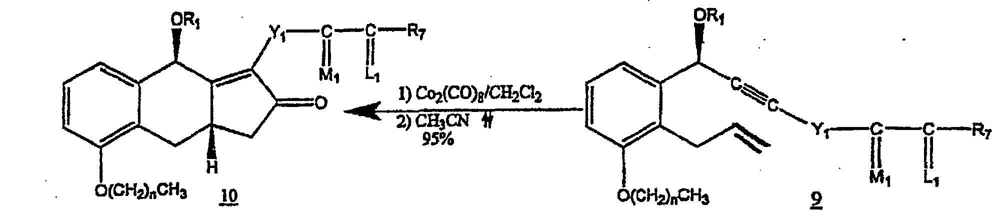 wobei der R₁ eine Alkohol-Schutzgruppe ist;
wobei n 0, 1, 2 oder 3 ist;
wobei Y₁ trans-CH=CH-, cis-CH=CH-, -CH₂(CH₂)ₘ- oder -C≡C- ist; m 1, 2 oder 3 ist;
wobei R₇ folgendes bedeutet
(1) -CₚH₂ₚ-CH₃, wobei p eine ganze Zahl von 1 bis einschließlich 5 ist;
(2) eine Phenoxygruppe, gegebenenfalls substituiert durch 1, 2 oder 3 Chloratome, Fluoratome, Trifluormethylgruppen, (C₁-C₃)-Alkylreste oder (C₁-C₃)-Alkoxyreste, mit der Maßgabe, daß nicht mehr als zwei Substituenten von einem Alkylrest verschieden sind, mit der Maßgabe, daß R₇ nur dann eine Phenoxygruppe oder substituierte Phenoxygruppe ist, wenn R₃ und R₄, gleich oder voneinander verschieden, ein Wasserstoffatom oder eine Methylgruppe sind;
(3) eine Phenyl-, Benzyl-, Phenylethyl- oder Phenylpropylgruppe, gegebenenfalls am aromatischen Ring durch 1, 2 oder 3 Chloratome, Fluoratome, Trifluormethylgruppen, (C₁-C₃)-Alkylreste oder (C₁-C₃)-Alkoxyreste substituiert, mit der Maßgabe, daß nicht mehr als zwei Substituenten von einem Alkylrest verschieden sind;
(4) cis-CH=CH-CH₂-CH₃;
(5) -(CH₂)₂-CH(OH)-CH₃ oder
(6) -(CH₂)₃-CH=C(CH₃)₂;
wobei -C(L₁)-R₇ zusammengenommen folgendes bedeutet:
(1) einen (C₄-C₇)-Cycloalkylrest, gegebenenfalls substituiert durch 1 bis 3 (C₁-C₅)-Alkylreste;
(2) eine 2-(2-Furyl)ethylgruppe;
(3) eine 2-(3-Thienyl)ethoxygruppe oder
(4) eine 3-Thienyloxymethylgruppe;
wobei M₁ α-OH:β-R₅ oder α- R₅:β-OH bedeutet, wobei R₅ ein Wasserstoffatom oder eine Methylgruppe ist;
wobei L₁ α-R₃:β- R₄, α-R₄:β-R₃ oder ein Gemisch von α-R₃:β-R₄ und α-R₄:β-R₃ ist,
wobei R₃ und R₄, gleich oder voneinander verschieden, ein Wasserstoffatom, eine Methylgruppe oder ein Fluoratom bedeuten, mit der Maßgabe, daß einer von R₃ und R₄ nur dann ein Fluoratom ist, wenn der andere ein Wasserstoff- oder Fluoratom ist.

2. Verfahren nach Anspruch 1, weiter umfassend die folgenden Schritte: 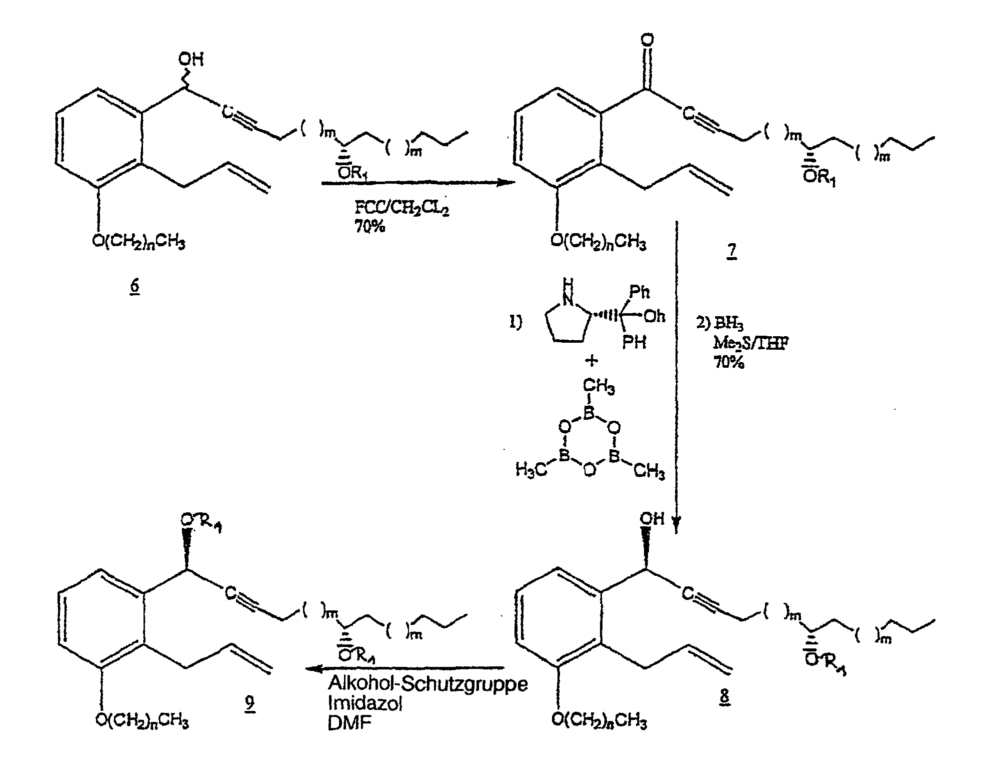 wobei R₁ eine unabhängig ausgewählte Alkohol-Schutzgruppe ist, m 1, 2 oder 3 ist und n 0, 1, 2 oder 3 ist.

3. Verfahren nach Anspruch 2, umfassend die folgenden Schritte: 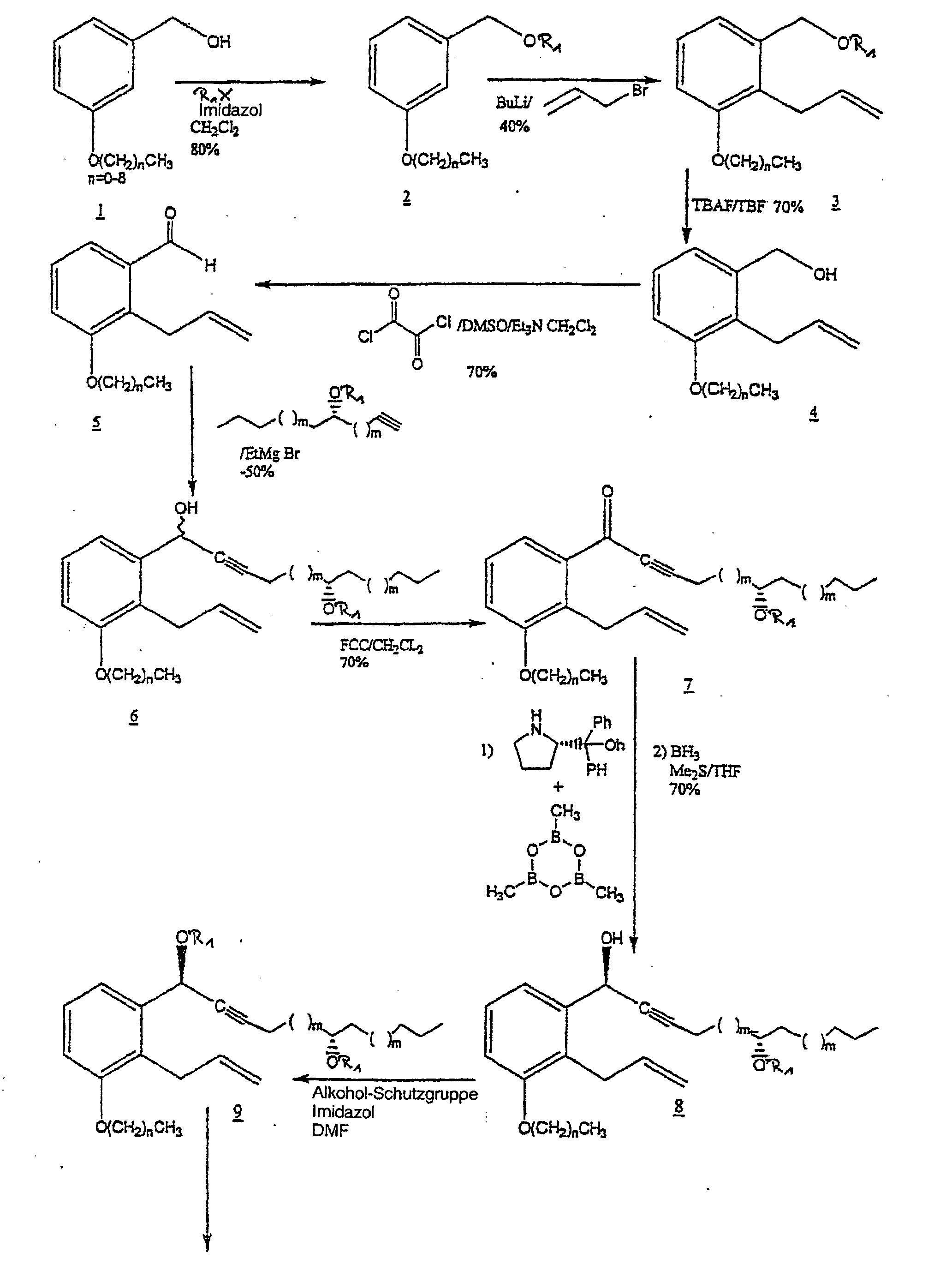 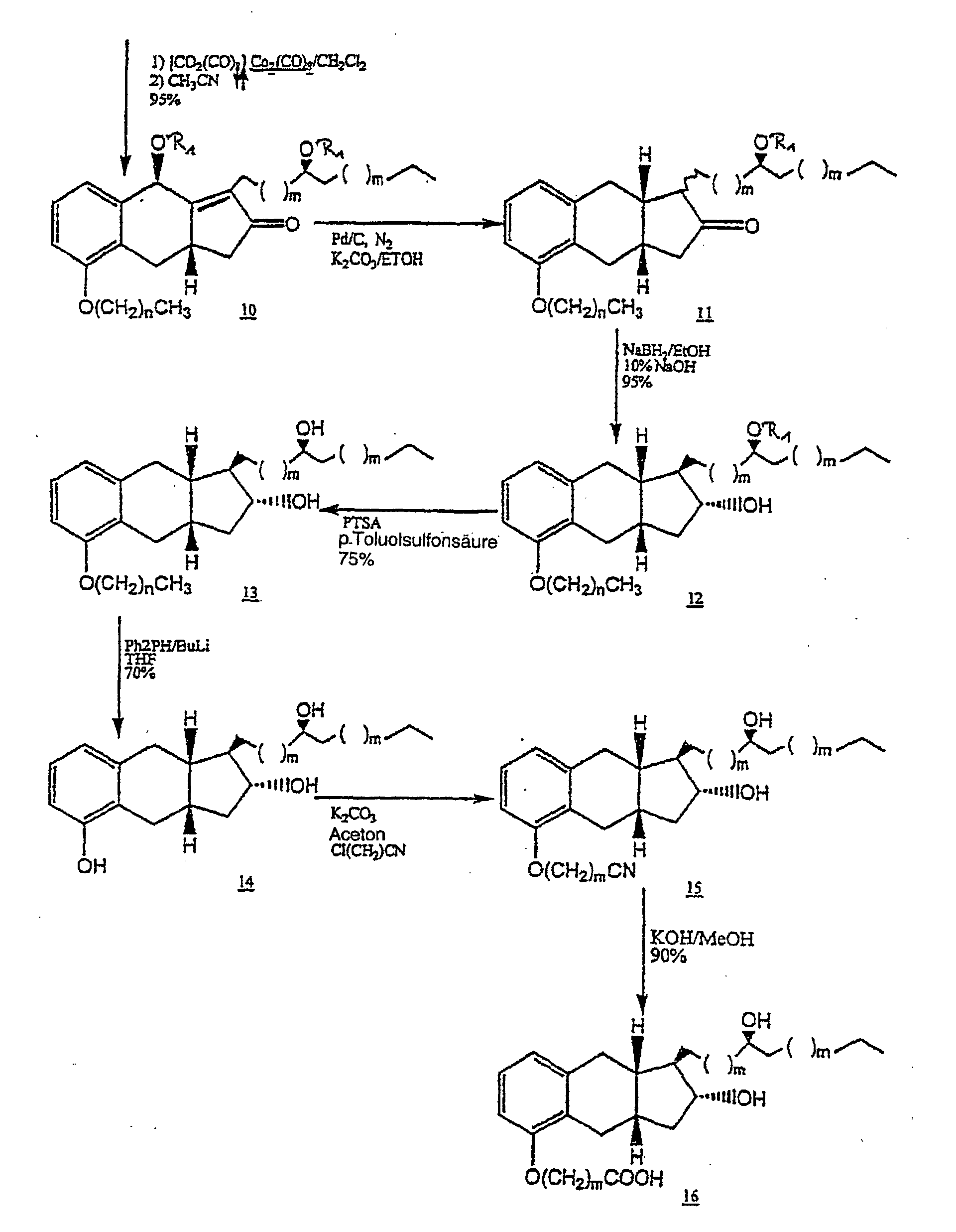 wobei R₁, m und n wie in Anspruch 2 definiert sind.

4. Verfahren nach Anspruch 3, wobei m 1 ist und n 0 ist.

5. Verbindung der Formel: 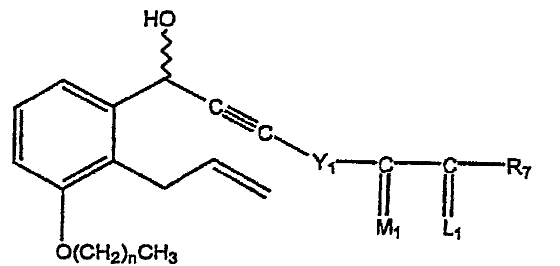 wobei Y₁ trans-CH=CH-, cis-CH=CH-, -CH₂(CH₂)ₘ- oder -C≡C- ist; m 1, 2 oder 3 ist;
wobei n 0, 1, 2 oder 3 ist;
wobei R₇ folgendes bedeutet:
(1) -CₚH₂ₚ-CH₃, wobei p eine ganze Zahl von 1 bis einschließlich 5 ist,
(2) eine Phenoxygruppe, gegebenenfalls substituiert durch 1, 2 oder 3 Chloratome, Fluoratome, Trifluormethylgruppen, (C₁-C₃)-Alkylreste oder (C₁-C₃)-Alkoxreste, mit der Maßgabe, daß nicht mehr als zwei Substituenten von einem Alkylrest verschieden sind, mit der Maßgabe, daß R₇ nur dann eine Phenoxygruppe oder substituierte Phenoxygruppe ist, wenn R₃ und R₄, die gleich oder verschieden sind, ein Wasserstoffatom oder eine Methylgruppe sind,
(3) eine Phenyl-, Benzyl-, Phenylethyl- oder Phenylpropylgruppe, gegebenenfalls am aromatischen Ring durch 1, 2 oder 3 Chloratome, Fluoratome, Trifluormethylgruppen, (C₁-C₃)-Alkylreste oder (C₁-C₃)-Alkoxyreste substituiert, mit der Maßgabe, daß nicht mehr als zwei Substituenten von einem Alkylrest verschieden sind,
(4) cis-CH=CH-CH₂-CH₃,
(5) -(CH₂)₂-CH(OH)-CH₃, oder
(6) -(CH₂)₃-CH=C(CH₃)₂;
wobei -C(L₁)-R₇ zusammengenommen für folgendes stehen:
(1) einen (C₄-C₇)-Cycloalkylrest, gegebenenfalls substituiert durch einen bis drei (C₁-C₅)-Alkylreste;
(2) eine 2-(2-Furyl)ethylgruppe;
(3) eine 2-(3-Thienyl)ethoxygruppe oder
(4) eine 3-Thienyloxymethylgruppe;
wobei M₁ für α-OH:β-R₅ oder α-R₅: β-OH steht, wobei R₅ ein Wasserstoffatom oder eine Methylgruppe bedeutet;
wobei L₁ für α-R₃:β-R₄, α-R₄:β-R₃ oder ein Gemisch von α-R₃:β-R₄ und α-R₄:β-R₃ steht, wobei R₃ und R₄, die gleich oder voneinander verschieden, ein Wasserstoffatom, eine Methylgruppe oder ein Fluoratom bedeuten, mit der Maßgabe, daß einer der Reste R₃ und R₄ nur dann ein Fluoratom ist, wenn der andere ein Wasserstoffatom oder Fluoratom ist.

6. Verbindung der Formel: 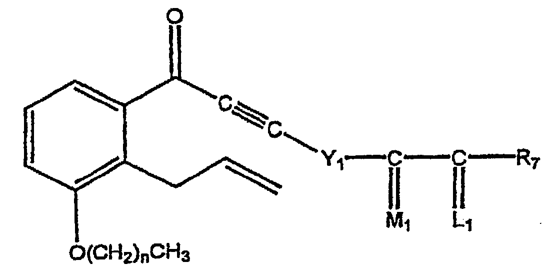 wobei Y₁ trans-CH=CH-, cis-CH=CH-, -CH₂(CH₂)ₘ- oder -C≡C- ist; m 1, 2 oder 3 ist;
wobei n 0, 1, 2 oder 3 ist;
wobei R₇ folgendes ist:
(1) -CₚH₂ₚ-CH₃, wobei p eine ganze Zahl von 1 bis einschließlich 5 ist,
(2) eine Phenoxygruppe, gegebenenfalls substituiert durch 1, 2 oder 3 Chloratome, Fluoratome, Trifluormethylgruppen, (C₁-C₃)-Alkylreste oder (C₁-C₃)-Alkoxyreste, mit der Maßgabe, daß nicht mehr als zwei Substituenten von einem Alkylrest verschieden sind, mit der Maßgabe, daß R₇ nur dann eine Phenoxygruppe oder substituierte Phenoxygruppe ist, wenn R₃ und R₄, gleich oder voneinander verschieden, ein Wasserstoffatom oder eine Methylgruppe sind,
(3) eine Phenylgruppe, Benzylgruppe, Phenylethylgruppe oder Phenylpropylgruppe, gegebenenfalls am aromatischen Ring durch 1, 2 oder 3 Chloratome, Fluoratome, Trifluormethylgruppen, (C₁-C₃)-Alkylreste oder (C₁-C₃)-Alkoxyreste substituiert, mit der Maßgabe, daß nicht mehr als zwei Substituenten von einem Alkylrest verschieden sind,
(4) cis-CH=CH-CH₂-CH₃,
(5) -(CH₂)₂-CH(OH)-CH₃ oder
(6) -(CH₂)₃-CH=C(CH₃)₂,
wobei -C(L₁)-R₇ zusammengenommen folgendes bedeutet:
(1) einen (C₄-C₇)-Cycloalkylrest, gegebenenfalls substituiert durch 1 bis 3 (C₁-C₅)-Alkylreste;
(2) eine 2-(2-Furyl)ethylgruppe,
(3) eine 2-(3-Thienyl)ethoxygruppe oder
(4) eine 3-Thienyloxymethylgruppe;
wobei M₁ α-OH:β-R₅ oder α-R₅:β-OH ist, wobei R₅ ein Wasserstoffatom oder eine Methylgruppe ist;
wobei L₁ α-R₃:β-R_{4,} α-R₄:β-R₃ oder ein Gemisch von α-R₃:β-R₄ und α-R₄:β-R₃ ist,
wobei R₃ und R₄, gleich oder voneinander verschieden, ein Wasserstoffatom, eine Methylgruppe oder ein Fluoratom sind, mit der Maßgabe, daß einer der Reste R₃ und R₄ nur dann ein Fluoratom ist, wenn der andere ein Wasserstoffatom oder ein Fluoratom ist.

7. Verbindung der Formel; 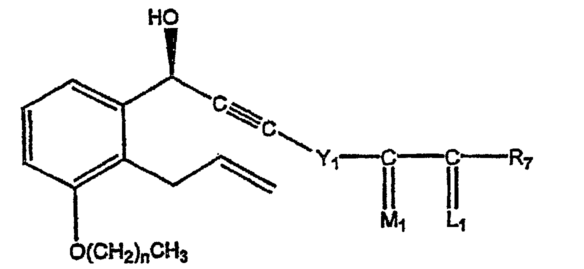 wobei Y₁ trans-CH=CH-, cis-CH=CH-, -CH₂(CH₂)ₘ- oder -C≡C- ist; m 1, 2 oder 3 ist;
wobei n 0, 1, 2 oder 3 ist;
wobei R₇ folgendes ist:
(1) -CₚH₂ₚ-CH₃, wobei p eine ganze Zahl von 1 bis einschließlich 5 ist,
(2) eine Phenoxygruppe, gegebenenfalls substituiert durch 1, 2 oder 3 Chloratome, Fluoratome, Trifluormethylgruppen, (C₁-C₃)-Alkylreste oder (C₁-C₃)-Alkoxyreste, mit der Maßgabe, daß nicht mehr als zwei Substituenten von einem Alkylrest verschieden sind, mit der Maßgabe, daß R₇ nur dann eine Phenoxygruppe oder substituierte Phenoxygruppe ist, wenn R₃ und R₄, gleich oder voneinander verschieden, ein Wasserstoffatom oder eine Methylgruppe sind,
(3) eine Phenylgruppe, Benzylgruppe, Phenylethylgruppe oder Phenylpropylgruppe, gegebenenfalls am aromatischen Ring durch 1, 2 oder 3 Chloratome, Fluoratome, Trifluormethylgruppen, (C₁-C₃)-Alkylreste oder (C₁-C₃)-Alkoxyreste substituiert, mit der Maßgabe, daß nicht mehr als zwei Substituenten von einem Alkylrest verschieden sind,
(4) cis-CH=CH-CH₂-CH₃,
(5) -(CH₂)₂-CH(OH)-CH₃ oder
(6) -(CH₂)₃-CH=C(CH₃)₂,
wobei -C(L₁)-R₇ zusammengenommen folgendes bedeutet:
(1) einen (C₄-C₇)-Cycloalkylrest, gegebenenfalls substituiert durch 1 bis 3 (C₁-C₅)-Alkylreste;
(2) eine 2-(2-Furyl)ethylgruppe,
(3) eine 2-(3-Thienyl)ethoxygruppe oder
(4) eine 3-Thienyloxymethylgruppe;
wobei M₁ α-OH:β-R₅ oder α-R₅:β-OH ist, wobei R₅ ein Wasserstoffatom oder eine Methylgruppe ist;
wobei L₁ α-R₃:β-R₄, α-R₄:β-R₃ oder ein Gemisch von α-R₃:β-R₄ und α-R₄:β-R₃ ist,
wobei R₃ und R₄, gleich oder voneinander verschieden, ein Wasserstoffatom, eine Methylgruppe oder ein Fluoratom sind, mit der Maßgabe, daß einer der Reste R₃ und R₄ nur dann ein Fluoratom ist, wenn der andere ein Wasserstoffatom oder ein Fluoratom ist.

8. Verbindung der Formel: 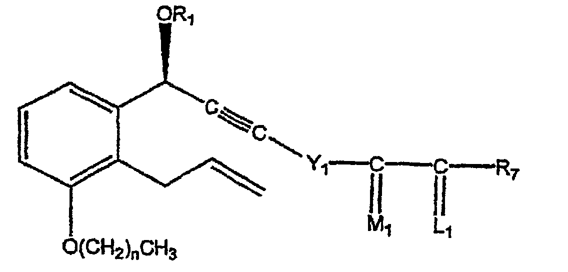 wobei R₁ eine Alkohol-Schutzgruppe ist;
wobei n 0, 1, 2 oder 3 ist;
wobei Y₁ trans-CH=CH-, cis-CH=CH-, -CH₂(CH₂)ₘ- oder -C≡C- ist; m 1, 2 oder 3 ist;
wobei R₇ folgendes ist:
(1) -CₚH₂ₚ-CH₃, wobei p eine ganze Zahl von 1 bis einschließlich 5 ist,
(2) eine Phenoxygruppe, gegebenenfalls substituiert durch 1, 2 oder 3 Chloratome, Fluoratome, Trifluormethylgruppen, (C₁-C₃)-Alkylreste oder (C₁-C₃)-Alkoxyreste, mit der Maßgabe, daß nicht mehr als zwei Substituenten von einem Alkylrest verschieden sind, mit der Maßgabe, daß R₇ nur dann eine Phenoxygruppe oder substituierte Phenoxygruppe ist, wenn R₃ und R₄, gleich oder voneinander verschieden, ein Wasserstoffatom oder eine Methylgruppe sind,
(3) eine Phenylgruppe, Benzylgruppe, Phenylethylgruppe oder Phenylpropylgruppe, gegebenenfalls am aromatischen Ring durch 1, 2 oder 3 Chloratome, Fluoratome, Trifluormethylgruppen, (C₁-C₃)-Alkylreste oder (C₁-C₃)-Alkoxyreste substituiert, mit der Maßgabe, daß nicht mehr als zwei Substituenten von einem Alkylrest verschieden sind,
(4) cis-CH=CH-CH₂-CH₃,
(5) -(CH₂)₂-CH(OH)-CH₃ oder
(6) -(CH₂)₃-CH=C(CH₃)₂,
wobei -C(L₁)-R₇ zusammengenommen folgendes bedeutet:
(1) einen (C₄-C₇)-Cycloalkylrest, gegebenenfalls substituiert durch 1 bis 3 (C₁-C₅)-Alkylreste;
(2) eine 2-(2-Furyl)ethylgruppe,
(3) eine 2-(3-Thienyl)ethoxygruppe oder
(4) eine 3-Thienyloxymethylgruppe;
wobei M₁ α-OH:β-R₅ oder α-R₅:β-OH ist, wobei R₅ ein Wasserstoffatom oder eine Methylgruppe ist;
wobei L₁ α-R₃;β-R₄, α-R₄:β-R₃ oder ein Gemisch von α-R₃:β-R₄ und α-R₄:β-R₃ ist,
wobei R₃ und R₄, gleich oder voneinander verschieden, ein Wasserstoffatom, eine Methylgruppe oder ein Fluoratom sind, mit der Maßgabe, daß einer der Reste R₃ und R₄ nur dann ein Fluoratom ist, wenn der andere ein Wasserstoffatom oder ein Fluoratom ist.

9. Verbindung der Formel 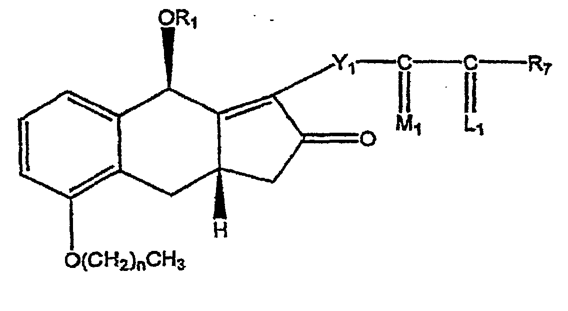 wobei R₁ eine Alkohol-Schutzgruppe ist;
wobei n 0, 1, 2 oder 3 ist;
wobei Y₁ trans-CH=CH-, cis-CH=CH-, -CH₂(CH₂)ₘ- oder -C≡C- ist; m 1, 2 oder 3 ist;
wobei R₇ folgendes ist:
(1) -CₚH₂ₚ-CH₃, wobei p eine ganze Zahl von 1 bis einschließlich 5 ist,
(2) eine Phenoxygruppe, gegebenenfalls substituiert durch 1, 2 oder 3 Chloratome, Fluoratome, Trifluormethylgruppen, (C₁-C₃)-Alkylreste oder (C₁-C₃)-Alkoxyreste, mit der Maßgabe, daß nicht mehr als zwei Substituenten von einem Alkylrest verschieden sind, mit der Maßgabe, daß R₇ nur dann eine Phenoxygruppe oder substituierte Phenoxygruppe ist, wenn R₃ und R₄, gleich oder voneinander verschieden, ein Wasserstoffatom oder eine Methylgruppe sind,
(3) eine Phenylgruppe, Benzylgruppe, Phenylethylgruppe oder Phenylpropylgruppe, gegebenenfalls am aromatischen Ring durch 1, 2 oder 3 Chloratome, Fluoratome, Trifluormethylgruppen, (C₁-C₃)-Alkylreste oder (C₁-C₃)-Alkoxyreste substituiert, mit der Maßgabe, daß nicht mehr als zwei Substituenten von einem Alkylrest verschieden sind,
(4) cis-CH=CH-CH₂-CH₃,
(5) -(CH₂)₂-CH(OH)-CH₃ oder
(6) -(CH₂)₃-CH=C(CH₃)₂,
wobei -C(L₁)-R₇ zusammengenommen folgendes bedeutet:
(1) einen (C₄-C₇)-Cycloalkylrest, gegebenenfalls substituiert durch ein bis drei (C₁-C₅)-Alkylreste;
(2) eine 2-(2-Furyl)ethylgruppe,
(3) eine 2-(3-Thienyl)ethoxygruppe oder
(4) eine 3-Thienyloxymethylgruppe;
wobei M₁ α-OH:β-R₅ oder α-R₅:β-OH ist, wobei R₅ ein Wasserstoffatom oder eine Methylgruppe ist;
wobei L₁ α-R₃:β-R_{4,} α-R₄:β-R₃ oder ein Gemisch von α-R₃:β-R₄ und α-R₄:β-R₃ ist,
wobei R₃ und R₄, gleich oder voneinander verschieden, ein Wasserstoffatom, eine Methylgruppe oder ein Fluoratom sind, mit der Maßgabe, daß einer der Reste R₃ und R₄ nur dann ein Fluoratom ist, wenn der andere ein Wasserstoffatom oder ein Fluoratom ist.

## Revendications

1. Procédé stéréosélectif de préparation d'un composé de type 9-désoxy-PGF₁, comprenant la réaction suivante : 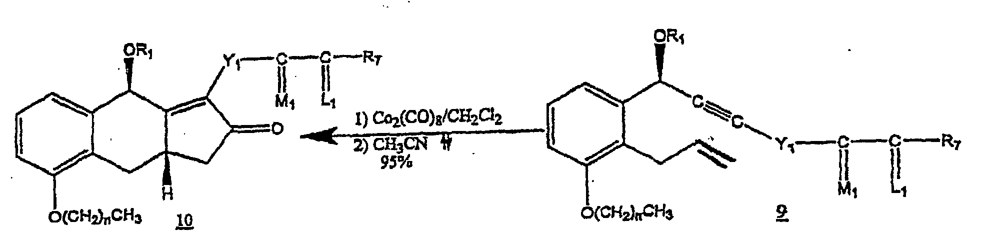 dans laquelle R₁ représente un groupe protecteur d'alcool ;
dans laquelle n est égal à 0, 1, 2, ou 3 ;
dans laquelle Y₁ représente un groupe -CH=CH- trans, -CH=CH-cis, -CH₂(CH₂)ₘ- ou -C≡C- ; m est égal à 1, 2, ou 3 ;
dans laquelle R₇ représente
(1) un groupe -CₚH₂ₚ-CH₃, où p représente un nombre entier de 1 à 5, bornes incluses,
(2) un groupe phénoxy éventuellement substitué par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle, à condition que R₇ représente un groupe phénoxy ou phénoxy substitué, seulement lorsque R₃ et R₄, identiques ou différents, représentent des atomes d'hydrogène ou des groupes méthyle,
(3) un groupe phényle, benzyle, phényléthyle ou phénylpropyle éventuellement substitué sur le cycle aromatique par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle,
(4) un groupe -CH=CH-CH₂-CH₃ cis,
(5) un groupe -(CH₂)₂-CH(OH)-CH₃, ou
(6) un groupe -(CH₂)₃-CH=C(CH₃)₂,
dans laquelle -C(L₁)-R₇ pris globalement représente
(1) un groupe cycloalkyle en C₄-C₇ éventuellement substitué par 1 à 3 groupes alkyle en C₁-C₅ ;
(2) un groupe 2-(2-furyl)éthyle,
(3) un groupe 2-(3-thiényl)éthoxy, ou
(4) un groupe 3-thiényloxyméthyle ;
dans laquelle M₁ représente un groupe α-OH:β-R₅ ou
α-R₅:β-OH, où R₅ représente un atome d'hydrogène ou un groupe méthyle ;
dans laquelle L₁ représente un groupe α-R₃:β-R₄, α-R₄:β-R₃, ou un mélange de α-R₃:β-R₄ et de α-R₄:β-R₃, où R₃ et R₄, identiques ou différents, représentent des atomes d'hydrogène, des groupes méthyle ou des atomes de fluor, à condition que l'un de R₃ et R₄ représente un atome de fluor seulement lorsque l'autre représente un atome d'hydrogène ou de fluor.

2. Procédé selon la revendication 1, comprenant en outre les étapes suivantes : 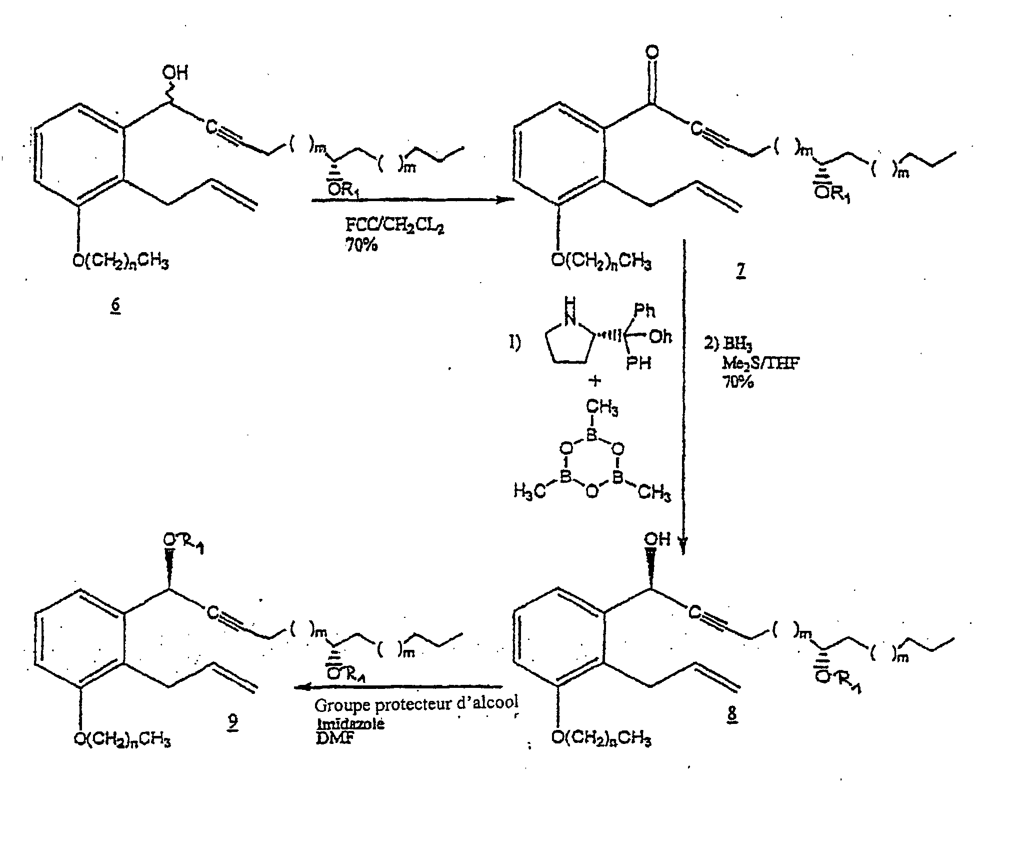 où R₁ représente un groupe protecteur d'alcool choisi indépendamment, m est égal à 1, 2, ou 3 et n est égal à 0, 1, 2, ou 3.

3. Procédé selon la revendication 2, comprenant les étapes suivantes : 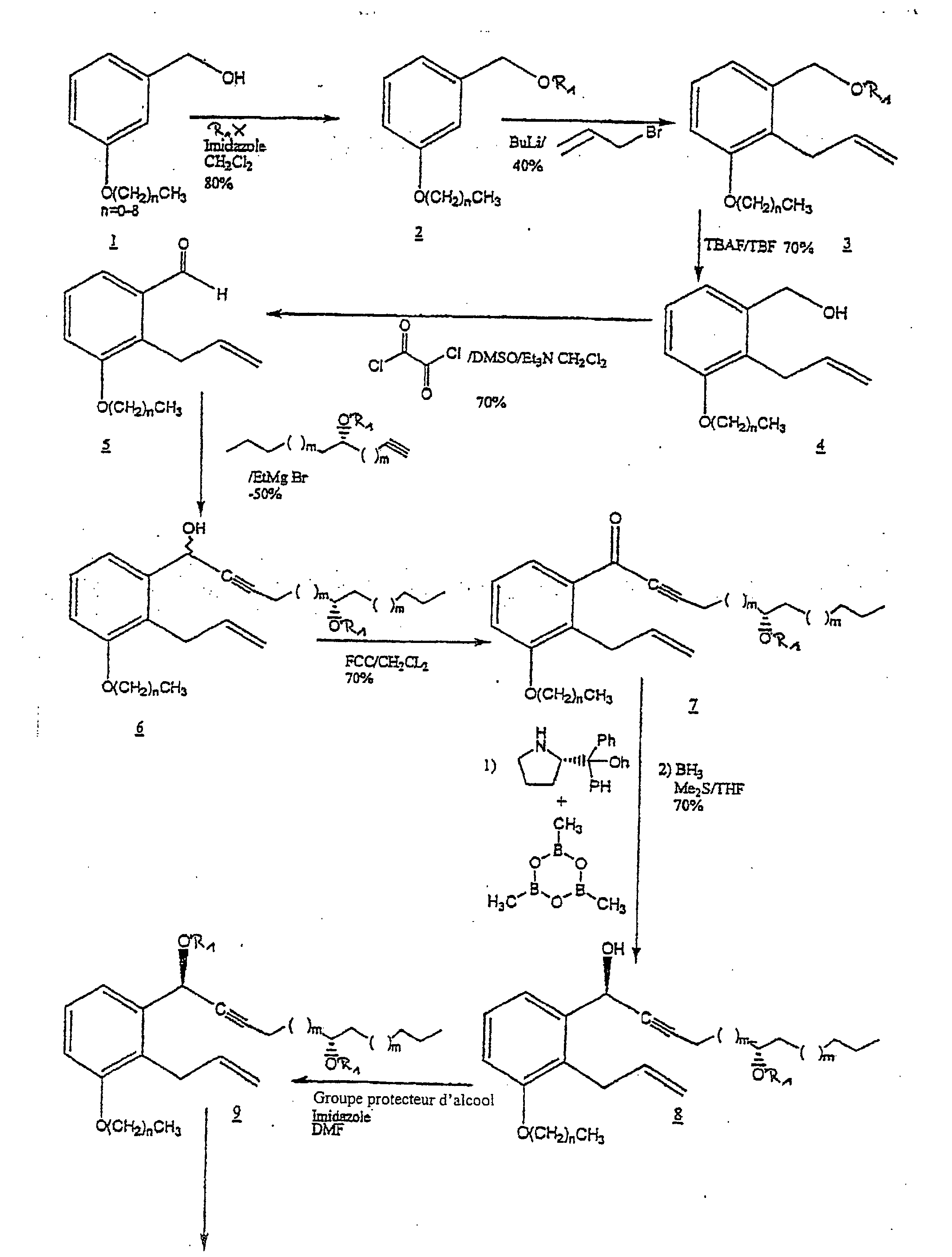 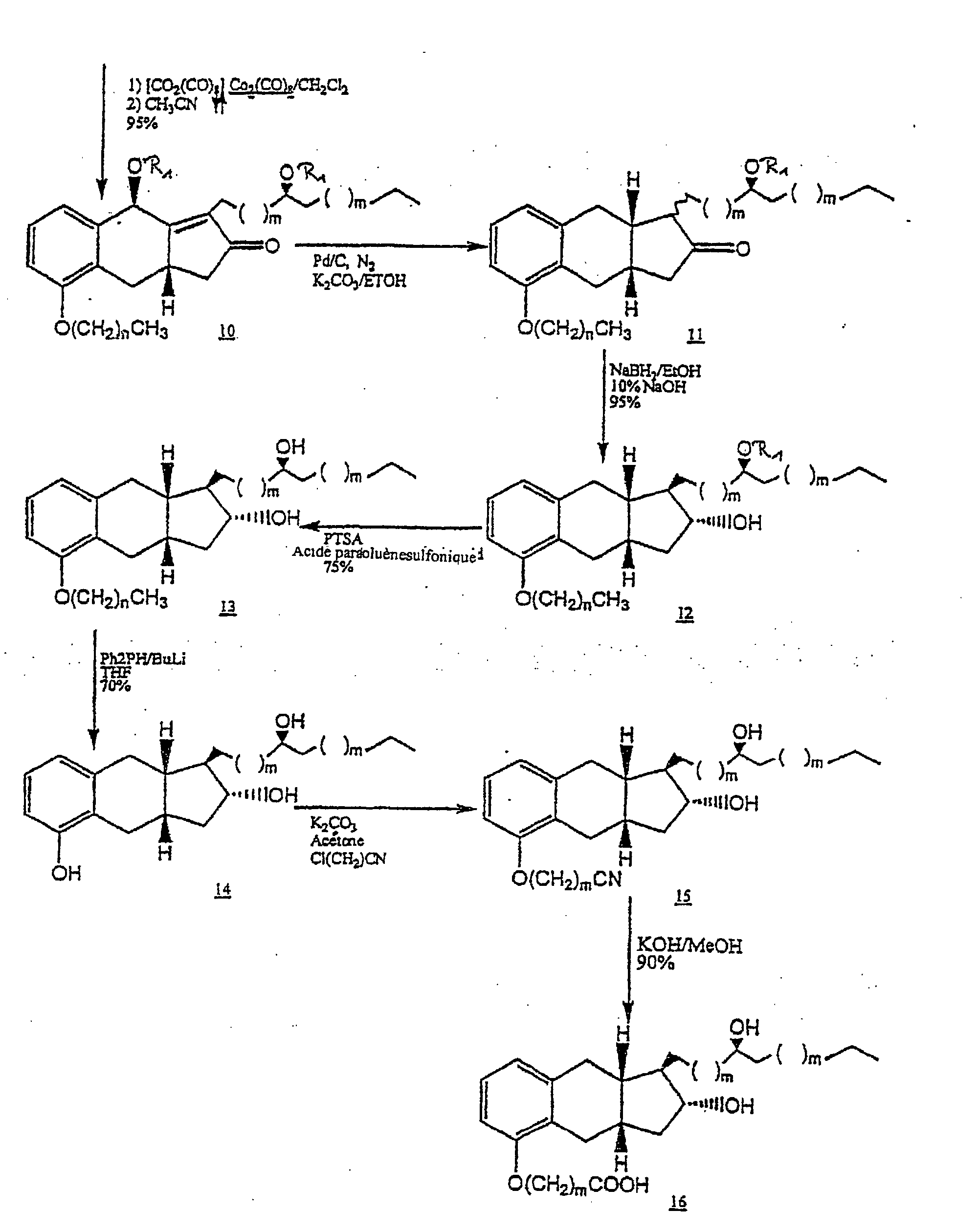 où R₁, m et n sont tels que définis dans la revendication 2.

4. Procédé selon la revendication 3 dans lequel m est égal à 1 et n est égal à 0.

5. Composé de formule : 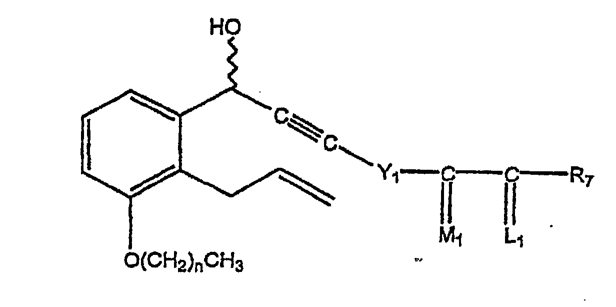 dans laquelle Y₁ représente un groupe -CH=CH- trans, -CH=CH- cis, -CH₂(CH₂)ₘ- ou -C≡C- ; m est égal à 1, 2, ou 3 ;
dans laquelle n est égal à 0, 1, 2, ou 3 ;
dans laquelle R₇ représente
(1) un groupe -CₚH₂ₚ-CH₃, où p représente un nombre entier de 1 à 5, bornes incluses,
(2) un groupe phénoxy éventuellement substitué par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle, à condition que R₇ représente un groupe phénoxy ou phénoxy substitué, seulement lorsque R₃ et R₄, identiques ou différents, représentent des atomes d'hydrogène ou des groupes méthyle,
(3) un groupe phényle, benzyle, phényléthyle ou phénylpropyle éventuellement substitué sur le cycle aromatique par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle,
(4) un groupe -CH=CH-CH₂-CH₃ cis,
(5) un groupe -(CH₂)₂-CH(OH)-CH₃, ou
(6) un groupe -(CH₂)₃-CH=C(CH₃)₂,
dans laquelle -C(L₁)-R₇ pris globalement représente
(1) un groupe cycloalkyle en C₄-C₇ éventuellement substitué par 1 à 3 groupes alkyle en C₁-C₅ ;
(2) un groupe 2-(2-furyl)éthyle,
(3) un groupe 2-(3-thiényl)éthoxy, ou
(4) un groupe 3-thiényloxyméthyle ;
dans laquelle M₁ représente un groupe α-OH:β-R₅ ou α-R₅:β-OH, où R₅ représente un atome d'hydrogène ou un groupe méthyle ;
dans laquelle L₁ représente un groupe α-R₃:β-R₄, α-R₄:β-R₃, ou un mélange de α-R₃:β-R₄ et de α-R₄:β-R₃, où R₃ et R₄, identiques ou différents, représentent des atomes d'hydrogène, des groupes méthyle ou des atomes de fluor, à condition que l'un de R₃ et R₄ représente un atome de fluor seulement lorsque l'autre représente un atome d'hydrogène ou de fluor.

6. Composé de formule : 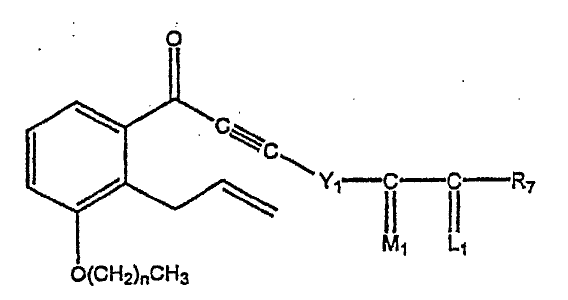 dans laquelle Y₁ représente un groupe -CH=CH- trans, -CH=CH- cis, -CH₂(CH₂)ₘ- ou -C≡C- ; m est égal à 1, 2, ou 3 ;
dans laquelle n est égal à 0, 1, 2, ou 3 ;
dans laquelle R₇ représente
(1) un groupe -CₚH₂ₚ-CH₃, où p représente un nombre entier de 1 à 5, bornes incluses,
(2) un groupe phénoxy éventuellement substitué par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle, à condition que R₇ représente un groupe phénoxy ou phénoxy substitué, seulement lorsque R₃ et R₄, identiques ou différents, représentent des atomes d'hydrogène ou des groupes méthyle,
(3) un groupe phényle, benzyle, phényléthyle ou phénylpropyle éventuellement substitué sur le cycle aromatique par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle,
(4) un groupe -CH=CH-CH₂-CH₃ cis,
(5) un groupe -(CH₂)₂-CH(OH)-CH₃, ou
(6) un groupe -(CH₂)₃-CH=C(CH₃)₂,
dans laquelle -C(L₁)-R₇ pris globalement représente
(1) un groupe cycloalkyle en C₄-C₇ éventuellement substitué par 1 à 3 groupes alkyle en C₁-C₅ ;
(2) un groupe 2-(2-furyl)éthyle,
(3) un groupe 2-(3-thiényl)éthoxy, ou
(4) un groupe 3-thiényloxyméthyle ;
dans laquelle M₁ représente un groupe α-OH:β-R₅ ou α-R₅:β-OH, où R₅ représente un atome d'hydrogène ou un groupe méthyle ;
dans laquelle L₁ représente un groupe α-R₃:β-R₄, α-R₄:β-R₃, ou un mélange de α-R₃:β-R₄ et de α-R₄:β-R₃, où R₃ et R₄, identiques ou différents, représentent des atomes d'hydrogène, des groupes méthyle ou des atomes de fluor, à condition que l'un de R₃ et R₄ représente un atome de fluor seulement lorsque l'autre représente un atome d'hydrogène ou de fluor.

7. Composé de formule : 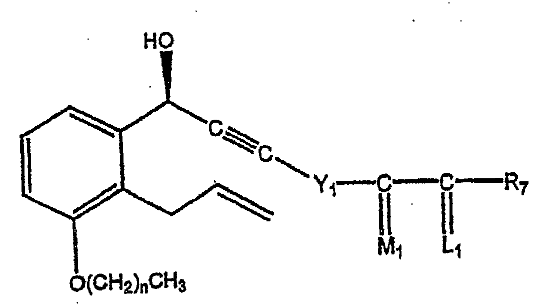 dans laquelle Y₁ représente un groupe -CH=CH- trans,
- CH=CH- cis, -CH₂(CH₂)ₘ- ou -C≡C- ; m est égal à 1, 2, ou 3 ;
dans laquelle n est égal à 0, 1, 2, ou 3 ; dans laquelle R₇ représente
(1) un groupe -CₚH₂ₚ-CH₃, où p représente un nombre entier de 1 à 5, bornes incluses,
(2) un groupe phénoxy éventuellement substitué par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle, à condition que R₇ représente un groupe phénoxy ou phénoxy substitué, seulement lorsque R₃ et R₄, identiques ou différents, représentent des atomes d'hydrogène ou des groupes méthyle,
(3) un groupe phényle, benzyle, phényléthyle ou phénylpropyle éventuellement substitué sur le cycle aromatique par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle,
(4) un groupe -CH=CH-CH₂-CH₃ cis,
(5) un groupe -(CH₂)₂-CH(OH)-CH₃, ou
(6) un groupe -(CH₂)₃-CH=C(CH₃)₂,
dans laquelle -C(L₁)-R₇ pris globalement représente
(1) un groupe cycloalkyle en C₄-C₇ éventuellement substitué par 1 à 3 groupes alkyle en C₁-C₅ ;
(2) un groupe 2-(2-furyl)éthyle,
(3) un groupe 2-(3-thiényl)éthoxy, ou
(4) un groupe 3-thiényloxyméthyle ;
dans laquelle M₁ représente un groupe α-OH:β-R₅ ou α-R₅:β-OH, où R₅ représente un atome d'hydrogène ou un groupe méthyle ;
dans laquelle L₁ représente un groupe α-R₃:β-R₄, α-R₄:β-R₃, ou un mélange de α-R₃:β-R₄ et de α-R₄:β-R₃, où R₃ et R₄, identiques ou différents, représentent des atomes d'hydrogène, des groupes méthyle ou des atomes de fluor, à condition que l'un de R₃ et R₄ représente un atome de fluor seulement lorsque l'autre représente un atome d'hydrogène ou de fluor.

8. Composé de formule : 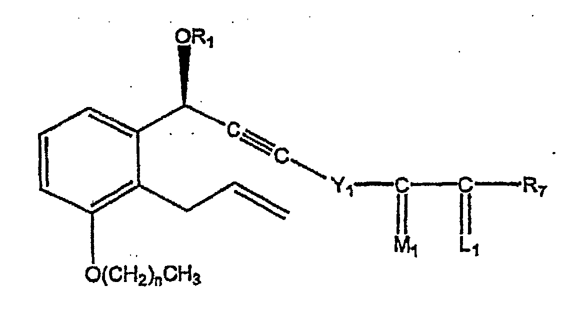 dans laquelle R₁ représente un groupe protecteur d'alcool ;
dans laquelle n est égal à 0, 1, 2, ou 3 ;
dans laquelle Y₁ représente un groupe -CH=CH- trans, -CH=CH-cis, -CH₂(CH₂)ₘ- ou -C≡C- ; m est égal à 1, 2, ou 3 ; dans laquelle R₇ représente
(1) un groupe -CₚH₂ₚ-CH₃, où p représente un nombre entier de 1 à 5, bornes incluses,
(2) un groupe phénoxy éventuellement substitué par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle, à condition que R₇ représente un groupe phénoxy ou phénoxy substitué, seulement lorsque R₃ et R₄, identiques ou différents, représentent des atomes d'hydrogène ou des groupes méthyle,
(3) un groupe phényle, benzyle, phényléthyle ou phénylpropyle éventuellement substitué sur le cycle aromatique par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle,
(4) un groupe -CH=CH-CH₂-CH₃ cis,
(5) un groupe -(CH₂)₂-CH(OH)-CH₃, ou
(6) un groupe -(CH₂)₃-CH=C(CH₃)₂,
dans laquelle -C(L₁)-R₇ pris globalement représente
(1) un groupe cycloalkyle en C₄-C₇ éventuellement substitué par 1 à 3 groupes alkyle en C₁-C₅ ;
(2) un groupe 2-(2-furyl)éthyle,
(3) un groupe 2-(3-thiényl)éthoxy, ou
(4) un groupe 3-thiényloxyméthyle;
dans laquelle M₁ représente un groupe α-OH:β-R₅ ou α-R₅:β-OH, où R₅ représente un atome d'hydrogène ou un groupe méthyle ;
dans laquelle L₁ représente un groupe α-R₃:β-R₄, α-R₄:β-R₃, ou un mélange de α-R₃:β-R₄ et de α-R₄:β-R₃, où R₃ et R₄, identiques ou différents, représentent des atomes d'hydrogène, des groupes méthyle ou des atomes de fluor, à condition que l'un de R₃ et R₄ représente un atome de fluor seulement lorsque l'autre représente un atome d'hydrogène ou de fluor.

9. Composé de formule : 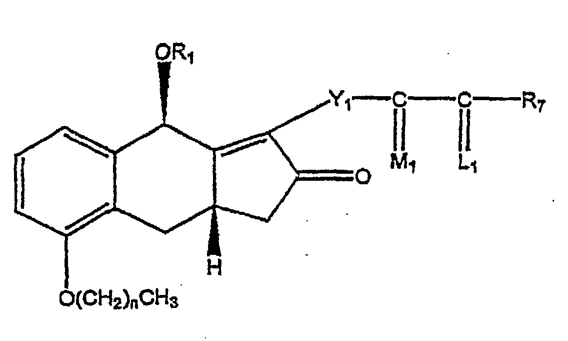 dans laquelle R₁ représente un groupe protecteur d'alcool ;
dans laquelle n est égal à 0, 1, 2, ou 3 ;
dans laquelle Y représente un groupe -CH=CH- trans, -CH=CH-cis, -CH₂(CH₂)ₘ- ou -C≡C- ; m est égal à 1, 2, ou 3 ;
dans laquelle R₇ représente
(1) un groupe -CₚH₂ₚ-CH₃, où p représente un nombre entier de 1 à 5, bornes incluses,
(2) un groupe phénoxy éventuellement substitué par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle, à condition que R₇ représente un groupe phénoxy ou phénoxy substitué, seulement lorsque R₃ et R₄, identiques ou différents, représentent des atomes d'hydrogène ou des groupes méthyle,
(3) un groupe phényle, benzyle, phényléthyle ou phénylpropyle éventuellement substitué sur le cycle aromatique par un, deux ou trois atomes de chlore, de fluor, groupes trifluorométhyle, alkyle en C₁-C₃, ou alcoxy en C₁-C₃, à condition que pas plus de deux substituants soient autres que des groupes alkyle,
(4) un groupe -CH=CH-CH₂-CH₃ cis,
(5) un groupe -(CH₂)₂-CH(OH)-CH₃, ou
(6) un groupe -(CH₂)₃-CH=C(CH₃)₂,
dans laquelle -C(L₁)-R₇ pris globalement représente
(1) un groupe cycloalkyle en C₄-C₇ éventuellement substitué par 1 à 3 groupes alkyle en C₁-C₅ ;
(2) un groupe 2-(2-furyl)éthyle,
(3) un groupe 2-(3-thiényl)éthoxy, ou
(4) un groupe 3-thiényloxyméthyle ;
dans laquelle M₁ représente un groupe α-OH:β-R₅ ou α-R₅:β-OH, où R₅ représente un atome d'hydrogène ou un groupe méthyle ;
dans laquelle L₁ représente un groupe α-R₃:β-R₄, α-R₄:β-R₃, ou un mélange de α-R₃:β-R₄ et de α-R₄:β-R₃, où R₃ et R₄, identiques ou différents, représentent des atomes d'hydrogène, des groupes méthyle ou des atomes de fluor, à condition que l'un de R₃ et R₄ représente un atome de fluor seulement lorsque l'autre représente un atome d'hydrogène ou de fluor.
